# EUROPEAN PATENT SPECIFICATION

(11) **EP 2 782 564 B1**
(45) Date of publication and mention of the grant of the patent: **09.01.2019**
(21) Application number: 12798116.5
(22) Date of filing: 21.11.2012
(51) Int. Cl.: A61K 31/145, A61K 45/06, A61P 7/02

(54) **CYSTEAMINE AND/OR CYSTAMINE FOR TREATING ISCHEMIC INJURY**
CYSTEAMIN UND/ODER CYSTAMIN ZUR BEHANDLUNG VON ISCHÄMIE
CYSTÉAMINE ET/OU CYSTAMINE POUR LE TRAITEMENT D'UNE LÉSION ISCHÉMIQUE

(30) Priority: 22.11.2011 US 201161563034 P
(43) Date of publication of application: 01.10.2014
(73) Proprietor: The Regents of the University of California, Oakland, CA 94607-5200 (US)
(72) Inventor: DOHIL, Ranjan, San Diego, CA 92130 (US); PHILLIPS, Susan, A., San Diego, CA 92122 (US)
(74) Representative: Daniels, Jeffrey Nicholas
(86) International application number: PCT/US2012/066288
(87) International publication number: WO 2013/078335

(56) References cited:
- EP-A2- 0 306 937
- US-A- 6 087 398
- US-A1- 2005 245 433
- DOHIL RANJAN ET AL: "The Effect of Cysteamine Bitartrate on Adiponectin Multimerization in Non-Alcoholic Fatty Liver Disease and Healthy Subjects", JOURNAL OF PEDIATRICS, vol. 161, no. 4, October 2012 (2012-10), page 639, XP002690824, ISSN: 0022-3476
- SHIN DONG-MYUNG ET AL: "Cystamine prevents ischemia-reperfusion injury by inhibiting polyamination of RhoA.", BIOCHEMICAL AND BIOPHYSICAL RESEARCH COMMUNICATIONS, vol. 365, no. 3, 18 January 2008 (2008-01-18), pages 509-514, XP002690825, ISSN: 1090-2104
- GUO ZHIXIN ET AL: "Effect of N-acetylcysteine on plasma adiponectin and renal adiponectin receptors in streptozotocin-induced diabetic rats.", EUROPEAN JOURNAL OF PHARMACOLOGY, vol. 558, no. 1-3, 8 March 2007 (2007-03-08), pages 208-213, XP002690826, ISSN: 0014-2999
- WANG TINGTING ET AL: "N-acetylcysteine and allopurinol synergistically enhance cardiac adiponectin content and reduce myocardial reperfusion injury in diabetic rats.", PLOS ONE, vol. 6, no. 8, E23967, August 2011 (2011-08), pages 1-11, XP002690827, ISSN: 1932-6203

## Description

### BACKGROUND

Acute ischemic stroke is estimated to affect ∼ 2-2.5 out of every thousand people, resulting in upwards of 4.5 million deaths per year worldwide and 9 million stroke survivors, with costs currently exceeding $50 billion in the U.S. alone.

### SUMMARY

The invention provides a cysteamine, cystamine, or a pharmaceutically acceptable salt of any of the foregoing for use in treating ischemia, ischemic injury, an acute ischemic event, thrombosis or an ischaemic injury resulting from thrombosis in a subject, wherein the use comprises increasing adiponectin levels in the subject and wherein the subject does not have diabetes or hypercholesterolemia. In an embodiment, the thrombosis is associated with sickle cell disease, deep vein thrombosis, pulmonary embolism, cardiac embolism, hypercoagulable state, thrombophilia, Factor V Leiden, Antithrombin III deficiency, Protein C deficiency, Protein S deficiency, Prothrombin gene mutation (G20210A), Hyperhomcysteinemia, antiphospholipid antibody syndrome (APS), anticardiolipin antibody (ACLA) thrombosis syndrome, or lupus anticoagulant (LA) syndrome. In any of the foregoing emeobidments, the amount of a cysteamine, cystamine, or a pharmaceutically acceptable salt thereof is effective to reduce the risk of ischemic injury resulting from thrombosis.

The disclosure also provides a method for treating a subject at risk of thrombosis and ischemic injury resulting therefrom comprising administering to the subject an amount of cysteamine, cystamine,
or a pharmaceutically acceptable salt of any of the foregoing, effective to reduce the risk of ischemic injury resulting from thrombosis.

In any of the foregoing embodiments, the subject may have cancer, a myeloproliferative disorder, multiple myeloma, may have had surgery or a trauma, may be immobilized, on oral contraceptive use, taking thalidomide or its congeners optionally in combination with a steroid, may have heparin-induced thrombocytopenia, may be pregnant, may have inflammatory bowel disease, a nephrotic syndrome, a paroxysmal nocturnal hemoglobinuria, a hyperviscosity syndrome, or Waldenstrom's macroglobulinemia.

The disclosure also provides a method for treating a subject at risk of thrombosis comprising administering to the subject an amount of a cysteamine, cystamine, or a pharmaceutically acceptable salt thereof in an amount effective to reduce the risk of ischemic injury due to thrombosis. In one embodiment, the subject has cancer, a myeloproliferative disorder, multiple myeloma, underwent surgery, had trauma, is under immobilization, taking an oral contraceptive, being administered thalidomide or its congeners optionally in combination with a steroid, has heparin-induced thrombocytopenia, is pregnant, has inflammatory bowel disease, has nephrotic syndrome, has paroxysmal nocturnal hemoglobinuria, has a hyperviscosity syndrome, or has Waldenstrom's macroglobulinemia.

In any of the foregoing embodiments, the ischemic injury can be the result of a tissue injury, a disease or a stroke. In a further embodiment, the injury is a reperfusion injury. In another embodiment, the reperfusion injury is a result of surgery or organ transplant. In another embodiment, the disease is Sickle Cell Disease.

In any of the foregoing embodiments, the cysteamine, cystamine, _ or a pharmaceutically acceptable salt thereof is administered within 72 hours after an ischemic event. In a further embodiment, the cysteamine, cystamine, or a pharmaceutically acceptable salt thereof is administered continuously after an ischemic event. In any of the foregoing embodiments, the cysteamine, cystamine, or a pharmaceutically acceptable salt thereof is administered continuously to a subject at risk of an ischemic event.

The disclosure also provides a method of increasing the ratio of low molecular weight (LMW) adiponectin levels to medium (MMW) and/or high molecular weight (HMW) adiponectin levels in a subject in need thereof comprising administering to the subject an amount of a cysteamine, cystamine, or a pharmaceutically acceptable salt thereof, effective to increase the ratio of low molecular weight adiponectin levels compared to a baseline prior to contacting the subject with the cysteamine, cystamine, or a pharmaceutically acceptable salt thereof, wherein the subject is not suffering from hypercholesterolemia or type II diabetes.

The disclosure also provides a method for treating a subject having irregular or abnormal ratios of high molecular weight (HMW):medium molecular weight (MMW) adiponectin, HMW:low molecular weight (LMW) adiponectin, or MMW:LMW adiponectin, comprising administering to the subject an amount of a cysteamine, cystamine, or a pharmaceutically acceptable salt thereof, effective to improve adiponectin ratios in the subject compared to a subject that did not receive the cysteamine, cystamine, or a pharmaceutically acceptable salt thereof.

In one embodiment of the foregoing the subject has a risk factor for ischemia selected from the group consisting of high blood pressure, tobacco use, carotid or other artery disease, peripheral artery disease, atrial fibrillation, other heart disease, transient ischemic attacks (TIAs), a blood disorders, physical inactivity, obesity, excessive alcohol use, illegal drug use, a prior stroke, Sickle Cell Anemia and prior heart attack. In another embodiment, the subject has previously suffered from an ischemic event. In any of the foregoing embodiments, the method comprises administering cysteamine, a or a pharmaceutically acceptable salt thereof. In a further embodiment, the cysteamine, cystamine, or a pharmaceutically acceptable salt of any of the foregoing, is administered at a concentration in the range of about 0.5-20 mg/kg of body weight of said subject. In yet a further embodiment of any of the foregoing, the total daily dose of the cysteamine, cystamine, or a pharmaceutically acceptable salt of any of the foregoing, is about 0.5-4.0 g/m². In yet still a further embodiment, the cysteamine, cystamine, or a pharmaceutically acceptable salt of any of the foregoing, is administered orally. In one embodiment, the cysteamine, cystamine or a pharmaceutically acceptable salt of any of the foregoing, is a delayed or controlled release dosage form. In any of the foregoing embodiments, the cysteamine, cystamine or a pharmaceutically acceptable salt of any of the foregoing, is administered intraperitoneally. In a further embodiment, the cysteamine, cystamine or a or a pharmaceutically acceptable salt of any of the foregoing, is administered intravenously or intra-arterially. In any of the foregoing embodiments, the administering results in a reduction in ischemia reperfusion injury or inflammation from an ischemic event. In another embodiment of any of the foregoing, the method further comprises administering one or more additional agents useful to treat ischemia. In a further embodiment, the one or more additional agents is selected from the group consisting of a reperfusion agent, a free-radical scavenger agent, and a spin trap agent, a neuroprotective agent, an anticoagulant, an antiplatelet agent, nimodipine, and naloxone. In another embodiment, the method further comprises administering one or more agents useful to treat a thrombotic disorder, optionally an anticoagulant, heparin, a vitamin K antagonist, 4-hydroxycoumarin derivatives, warfarin, acenocoumarol, dicumarol, ethyl biscoumacetate, phenprocoumon, streptokinase, urokinase, tissue plasminogen activator (tPA), alteplase (recombinant tPA), reteplase, tenecteplase, and argatroban. In yet another embodiment, the cysteamine, cystamine or or a pharmaceutically acceptable salt of any of the foregoing, is administered less than four times per day. In yet a further embodiment, the cysteamine, cystamine is administered twice daily. In any of the foregoing embodiments, the cysteamine, cystamine, or a pharmaceutically acceptable salt thereof is administered continuously for a period of days, weeks or months.

The disclosure provides a method of treating an ischemic event comprising contacting a subject suffering from an ischemic event with an effective amount of a cysteamine, cystamine, or a pharmaceutically acceptable salt thereof, wherein the subject has improved behavioral outcome compared to a subject that did not receive the cysteamine, cystamine, or a pharmaceutically acceptable salt thereof. In one embodiment, the subject's adiponectin levels are increased following contacting with the cysteamine, cystamine, or a pharmaceutically acceptable salt thereof. In yet another embodiment, the total daily dose of the cysteamine, cystamine, or a pharmaceutically acceptable salt thereof is about 0.5-4.0 g/m². In yet a further embodiment, the cysteamine, cystamine, or a pharmaceutically acceptable salt thereof is administered intraperitoneally. In yet another embodiment, the cysteamine, cystamine, or a pharmaceutically acceptable salt thereof is administered orally. In a further embodiment, the cysteamine, cystamine, or a pharmaceutically acceptable salt thereof is a delayed or controlled release dosage form that provides increased delivery of the cysteamine, cystamine, or a pharmaceutically acceptable salt thereof to the small intestine. In yet a further embodiment, the delayed or controlled release dosage form comprises an enteric coating that releases the cysteamine, cystamine, or a pharmaceutically acceptable salt thereof when the cysteamine, cystamine, or a pharmaceutically acceptable salt thereof reaches the small intestine or a region of the gastrointestinal tract of a subject in which the pH is greater than about pH 4.5. In one embodiment, the enteric coating is a coating selected from the group consisting of polymerized gelatin, shellac, methacrylic acid copolymer type C NF, cellulose butyrate phthalate, cellulose hydrogen phthalate, cellulose proprionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and acrylic acid polymers and copolymers, typically formed from methyl acrylate, ethyl acrylate, methyl methacrylate and/or ethyl methacrylate with copolymers of acrylic and methacrylic acid esters. In yet another embodiment, when the cysteamine, cystamine, or a pharmaceutically acceptable salt thereof is administered orally, the cysteamine, cystamine, or a pharmaceutically acceptable salt thereof is administered with a bioavailable iron formulation. In any of the foregoing embodiment, the administering results in a reduction in inflammation from the ischemic event.

In accordance with the invention, the administration of the cysteamine, cystamine, or a pharmaceutically acceptable salt thereof to the subject increases adiponectin levels compared to a baseline prior to contacting the subject with the cysteamine, cystamine, or a pharmaceutically acceptable salt of any of the foregoing. In one embodiment, the total adiponectin levels are increased. In another embodiment, the ratio of lower molecular weight adiponectin to medium and/or high molecular weight adiponectin is increased. In yet another embodiment, the subject is obese and has low LMW adiponectin levels compared to healthy subjects. In one embodiment, the subject has diabetes and has low LMW adiponectin levels compared to healthy subjects. In yet another embodiment, the subject suffers from ischemic events.

The invention provides a cysteamine, cystamine, or a pharmaceutically acceptable salt thereof for use in treating ischemia, ischemic injury, an acute ischemic event, thrombosis or ischemic injury resulting from thrombosis. In one embodiment, the ischemia or ischemic injury is the result of a thrombotic disorder. In another embodiment, the thrombotic disorder is sickle cell disease, deep vein thrombosis, pulmonary embolism, hypercoagulable state, thrombophilia, Factor V Leiden, Antithrombin III deficiency, Protein C deficiency, Protein S deficiency, Prothrombin gene mutation (G20210A), Hyperhomcysteinemia, antiphospholipid antibody syndrome (APS), anticardiolipin antibody (ACLA) thrombosis syndrome, or lupus anticoagulant (LA) syndrome. In yet another embodiment, the cysteamine, cystamine, or a pharmaceutically acceptable salt thereof is cysteamine, or a pharmaceutically acceptable salt thereof. In yet a further embodiment, the ischemia is caused by a risk factor selected from the group consisting of high blood pressure, tobacco use, carotid or other artery disease, peripheral artery disease, atrial fibrillation, other heart disease, transient ischemic attacks (TIAs), a blood disorders, high blood cholesterol, physical inactivity, obesity, excessive alcohol use, illegal drug use, a prior stroke, Sickle Cell Anemia and prior heart attack. In another embodiment, the cysteamine, cystamine, or a pharmaceutically acceptable salt thereof is formulated for intraperitoneal administration. In any of the foregoing embodiments, the cysteamine, cystamine, or a pharmaceutically acceptable salt thereof is administered intravascularly. In yet a further embodiment of any of the foregong, the cysteamine, cystamine, or a pharmaceutically acceptable salt thereof is formulated for oral administration. In a further embodiment, the cysteamine, cystamine or a pharmaceutically acceptable salt thereof is enterically coated. In any of the foregoing embodiments the cysteamine, cystamine, or a pharmaceutically acceptable salt thereof is administered continuously. In another embodiment, the cysteamine, cystamine, or a pharmaceutically acceptable salt thereof is administered prior to, simultaneously with or after an ischemic event/crisis. In yet another embodiment, the cysteamine, cystamine, or a pharmaceutically acceptable salt thereof is used to increase adiponectin levels in the subject.

The details of one or more embodiments of the disclosure are set forth in the accompanying drawings and the description below. Other features, objects, and advantages will be apparent from the description and drawings, and from the claims.

### BRIEF DESCRIPTION OF THE FIGURES

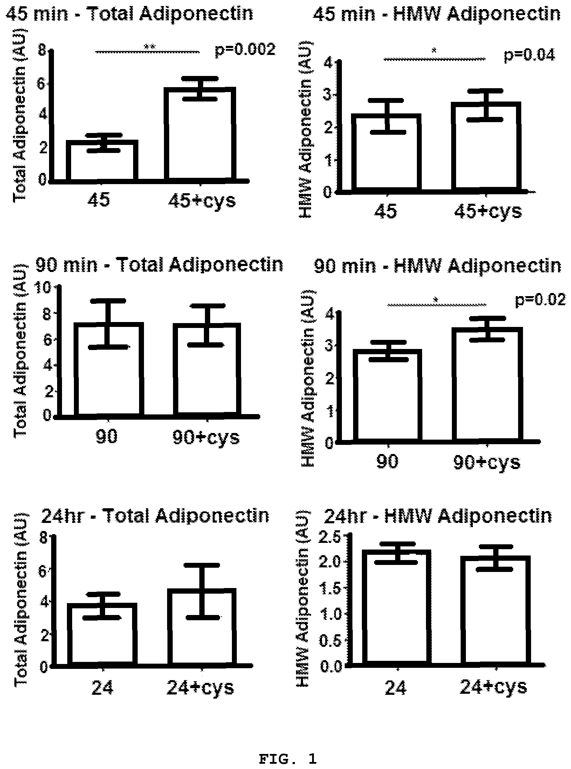

**Figure 1** shows treatment of adipose tissue explants with 90µM cysteamine for 45 min., 90 min., and 24hrs. Significant difference in total adiponectin with 90 µM cysteamine treatment at 45 min.: p=0.002; significant difference in HMW adiponectin with 90 µM cysteamine treatment at 45 min.: p=0.04; significant difference in HMW adiponectin with 90 µM cysteamine treatment at 90 min.: p=0.02.

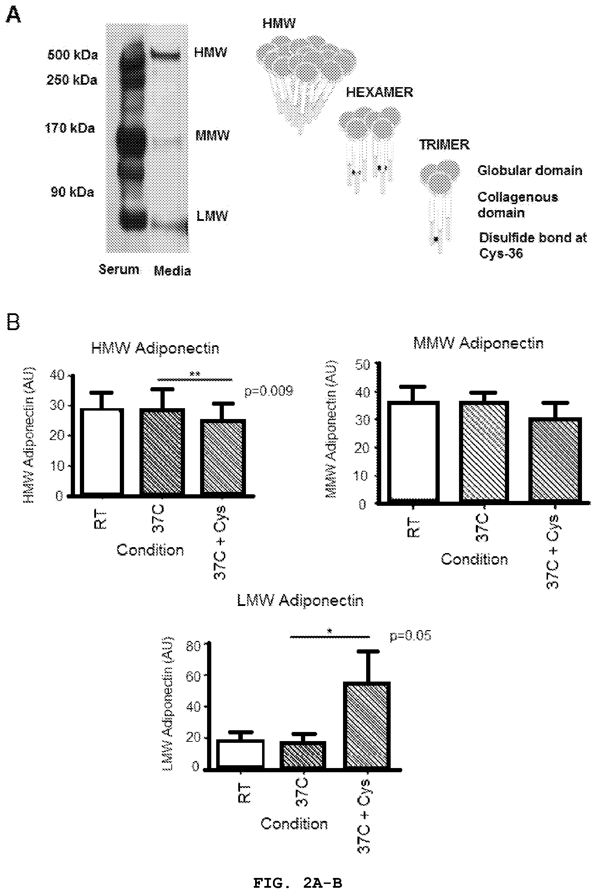

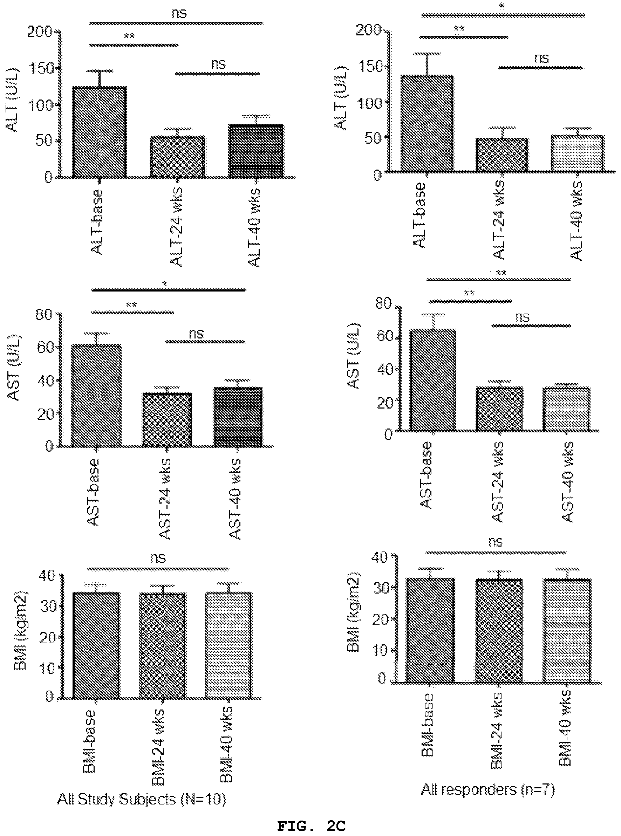

**Figure 2A-C** shows (A) a schematic and relative mobility shift of LMW, MMW and HMW adiponectin; (B) shows Gastric Banding (GB) serum incubated at room temperature, 37°C, 37°C +45µM cysteamine for 1hr and the production of adiponectin multimers. No significant difference between room temperature and 37°C for any multimer was observed. However: HMW significantly decrease with cysteamine treatment: p=0.009; MMW had significant change with cysteamine treatment; and LMW significantly increase with cysteamine treatment: p=0.05. (C) Mean values for BMI, ALT, and AST measured at baseline, after 24 weeks of treatment with EC-cysteamine and then 16 weeks after discontinuing EC-cysteamine). The P values are denoted as not significant (ns), *P < .05, **P < .01. Mean BMI was not significantly different at any timepoints. SEM is shown.

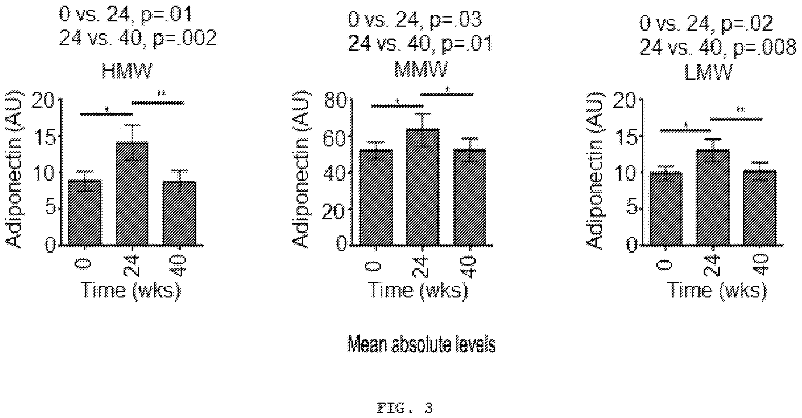

**Figure 3** shows *in vivo* effects of cysteamine on adiponectin multimerization in subjects with NAFLD. Eleven subjects with NAFLD were treated with oral enteric-coated cysteamine for 24 weeks and then monitored without treatment for 24 weeks. Serum was analysed at baseline, 24 weeks on treatment and then 24 weeks later off treatment. There was a significant increase in total and also in all sub-forms of adiponectin following 24 weeks of oral EC-cysteamine followed by a significant reduction 24 weeks after discontinuing therapy.

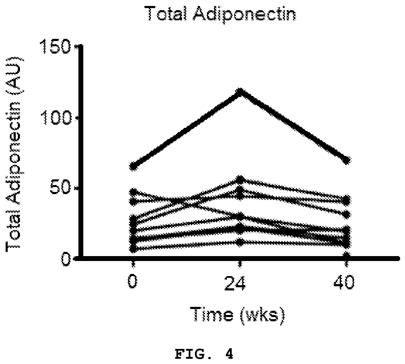

**Figure 4** shows total adiponectin with *in vivo* cysteamine treatment. Total adiponectin was significantly increased from 0-24 weeks: p=0.05; with a significant decrease in absolute level from 24-40 weeks: p=0.01; No significant difference between baseline and 40wks.

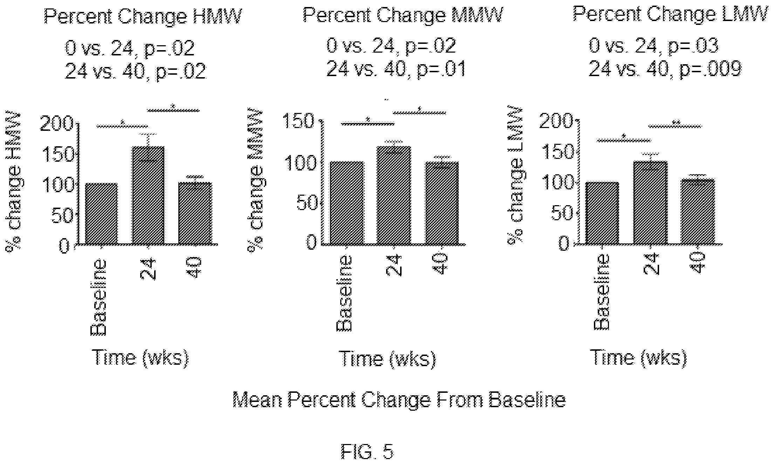

**Figure 5** shows similar data as Figure 3, but expressed as mean percent change of the *in vivo* effects of cysteamine on adiponectin multimerization. Serum from subjects at time 0 (basal), 24 weeks of treatment, and at 40 weeks following discontinuation of cysteamine at 24 weeks. Semi-quantitative analysis performed by western blot on 1ul of serum under non-reduced conditions. Subject data normalized using % change of adiponectin multimers at 24 weeks versus baseline and at 40 weeks versus baseline.

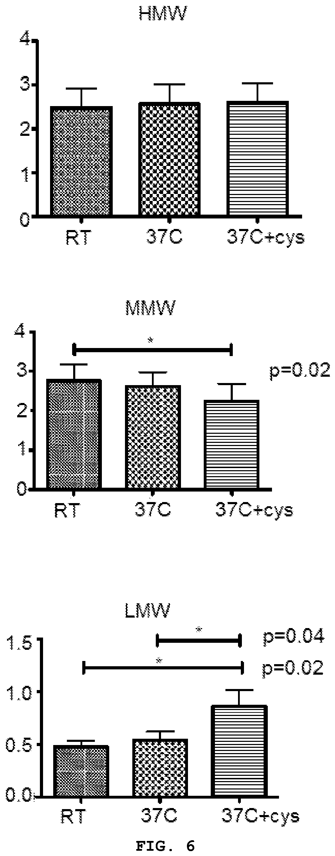

**Figure 6** shows *in vitro* effects of cysteamine on lean control subjects' absolute adiponectin levels. Lean control serum at room temperature, 37°C, 37°C + 90µM cysteamine were incubate for 1hr. No significant difference between room temperature and 37°C for any multimer HMW no significant change with cysteamine treatment; MMW significant decrease in absolute level with cysteamine treatment; LMW no significant change in absolute level with cysteamine treatment (data trends toward a significant increase) significant % change with cysteamine treatment.

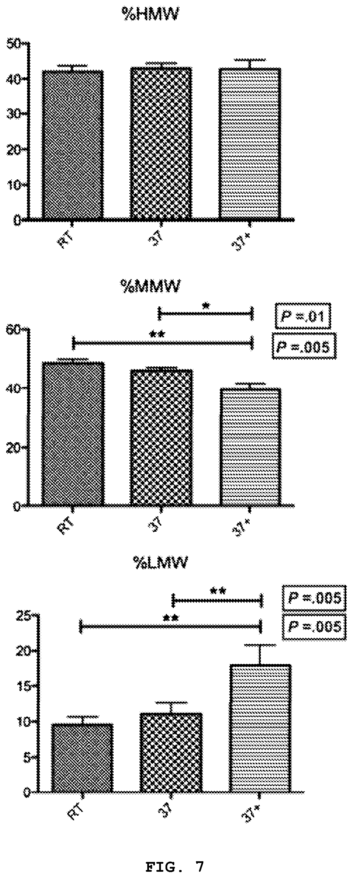

**Figure 7** shows lean control serum at room temperature, 37°C, 37°C + 90µM cysteamine following 1hr incubation. No significant difference between room temperature and 37°C for any multimer. HMW showed no significant change with cysteamine treatment. MMW showed significant decrease in absolute level with cysteamine treatment; a significant percent change with cysteamine treatment; and LMW showed no significant change in absolute level with cysteamine treatment (data trends toward a significant increase) significant percent change with cysteamine treatment.

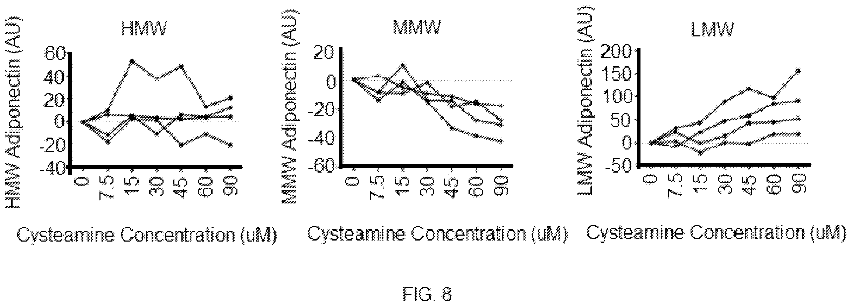

**Figure 8** are graphs of *in vitro* effect of cysteamine on serum taken from gastric banding subjects. Cysteamine concentrations 0, 7.5, 15, 30, 45, 60, 90µM with 1hr incubation at 37°C.

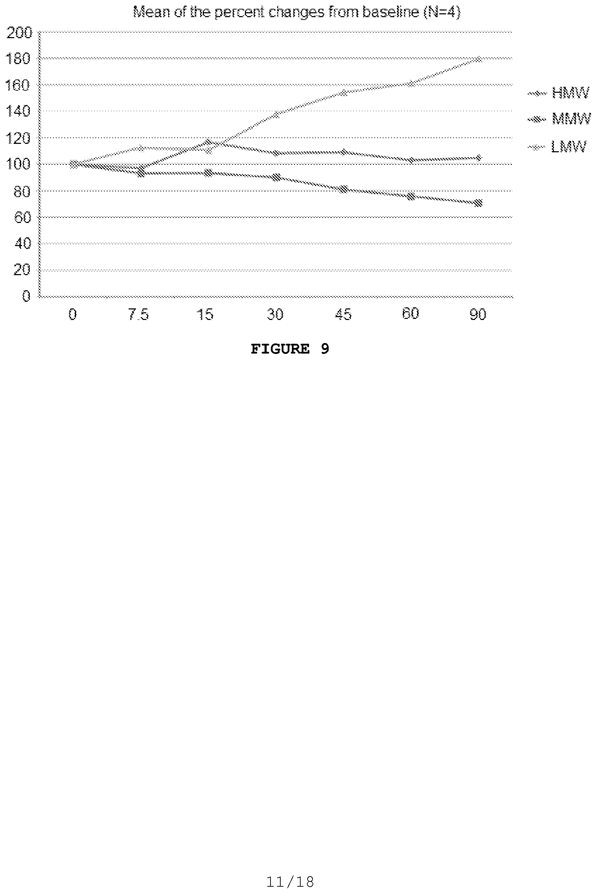

**Figure 9** shows adiponectin levels measured after one hour following addition of cysteamine (at different concentrations: 0, 7.5, 15, 30, 45, 60 and 90µM) to serum from 4 patients with obesity.

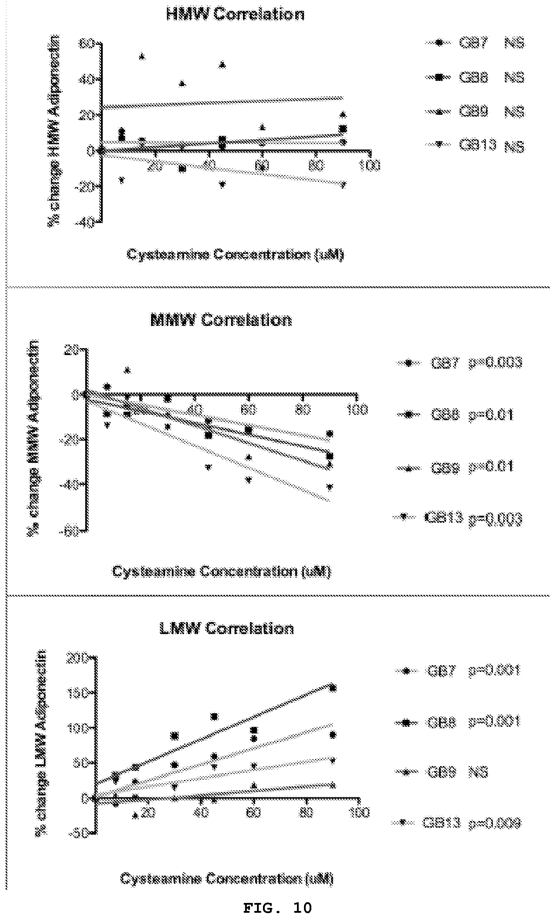

**Figure 10** shows percent change of different adiponectin multimers at various concentrations of cysteamine.

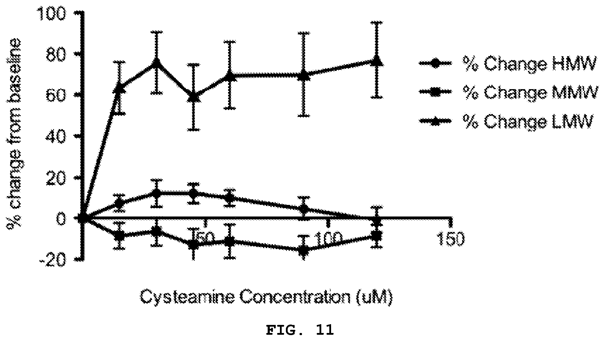

**Figure 11** shows mean percent change from baseline for adiponectin forms following incubation of BAT1-16 serum with different concentration of cysteamine for 1 hour.

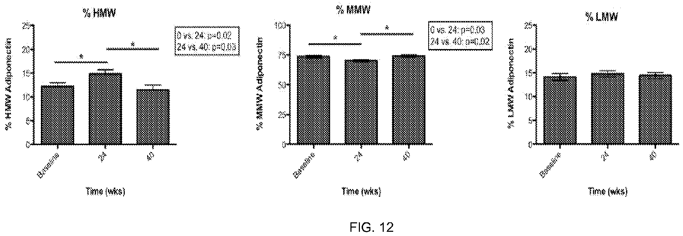

**Figure 12** shows levels of all multimer forms increased following 24wks cysteamine therapy. However, the relative proportions of each multimer that made up total adiponectin increased significantly for HMW, decreased for MMW and remained unchanged for LMW.

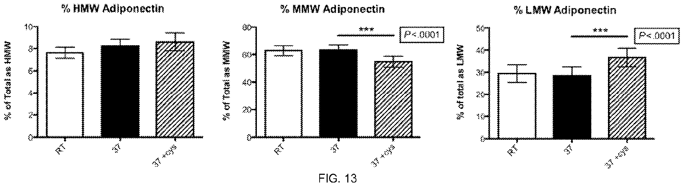

**Figure 13** shows mean percent changes in adiponectin multimers in subjects (n=10) with NAFLD. There was no significant difference for any multimer measured between room temperature (RT open bars) and 37°C (solid bars). In serum incubated with cysteamine at 45µM at 37°C for one hour (hatched bars) a significant reduction in MMW and increase in LMW was observed when compared with untreated serum.

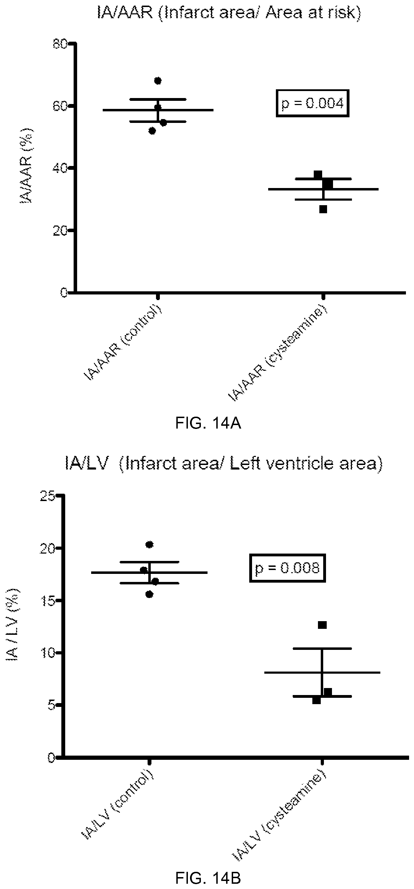

**Figure 14A-B** shows *in vivo* effects of cysteamine. (A) is a graph showing a significant difference between cysteamine treated and control for ischemic area as a percentage of the area at risk (or region of myocardium supplied by LAD artery. (B) is a graph showing a significant difference between cysteamine treated and control group for ischemic area as a percentage of the left ventricular area.

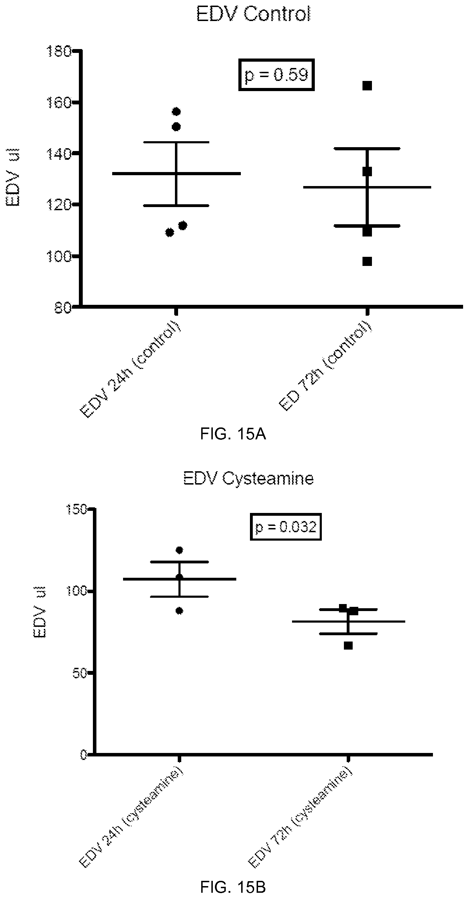

**Figure 15A-B** shows diastolic volumes in cysteamine treated and untreated models. (A) shows the end diastolic volume in µL at 24 hours and 72 hours in control group receiving IV buffer alone. There was no significant difference in EDV between the two time points suggesting that there was no improvement over time. (B) shows the end diastolic volume in µL at 24 hours and 72 hours in control group receiving cysteamine in IV buffer. There was a significant reduction in EDV between the two time points suggesting that there was improvement over time.

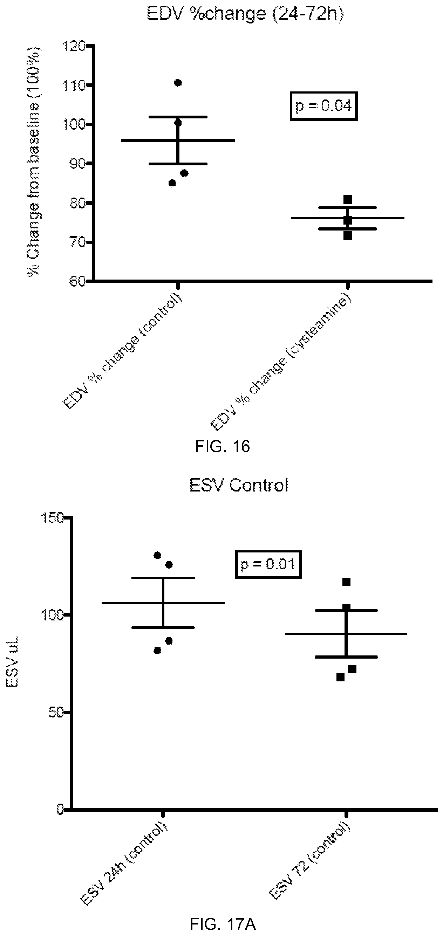

**Figure 16** is a graph showing that there was a significant difference between the percent change in EDV between 24h and 72h. A reduction in EDV suggests less ventricular dilation following the myocardial ischemia. The mice dropped weight during between 24h and 72h and were lethargic. The control mice lost 13% and the cysteamine group lost 14% body weight).

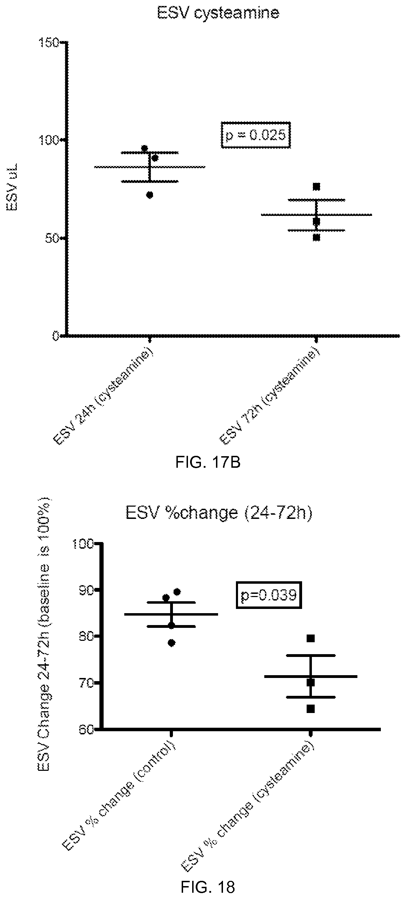

**Figure 17A-B** shows graphs of end systolic volume (ESV) for control and cysteamine treated animal models of ischemia. (A) shows ESV in µL at 24 hours and 72 hours in control group receiving IV buffer alone. There was significant difference in ESV. (B) shows ESV in µL at 24 hours and 72 hour in treatment group receiving IV cysteamine in buffer. There was significant difference in ESV.
**Figure 18** shows a graph as a percent change in ESV. There was a significant difference between the percent change in ESV between 24 hour and 72 hours. A reduction in ESV suggests better ventricular contraction.

### DETAILED DESCRIPTION

As used herein and in the appended claims, the singular forms "a," and "the" include plural referents unless the context clearly dictates otherwise. Thus, for example, reference to "an antioxidant" includes a plurality of such antioxidants and reference to "the antioxidant" includes reference to one or more antioxidants and so forth.

Similarly, "comprise," "comprises," "comprising" "include," "includes," and "including" are interchangeable.

It is to be further understood that where descriptions of various embodiments use the term "comprising," those skilled in the art would understand that in some specific instances, an embodiment can be alternatively described using language "consisting essentially of" or "consisting of."

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

As used herein ischemia refers to a condition resulting from a decrease or lack of blood flow and oxygen to a part of the body such as the brain, heart, or other tissue. Ischemic injury refers generally to the damage to a tissue that is distal or otherwise effected by the loss of blood flow and oxygen. Ischemic injury is often a result of the lack of oxygen and fluids, but also includes inflammatory cascades. For example, ischemia and ischemic injury can occur as a result of cardiac, pulmonary or brain injury, organ transplantation or surgical procedure, or a disease or disorder (e.g., Sickle Cell Anemia or Sickle Cell Disease).

Acute ischemia is most often recognized in strokes and cardiac damage. However, there are a number of disorders and injuries that cause ischemic events leading to cell death and tissue damage. For example, heart attacks (*i.e*., myocardial infarction) are common. Also someone who has evidence of intermittent milder cardiac ischemia (*i.e*., angina) may benefit from cysteamine (possibly through its long-term adponectin effect). Strokes, cerebrovascular events and cardio vascular events are the result of an acute obstruction of cerebral or cardiac blood flow to a region of the brain or heart, respectively. There are approximately 500,000 cases of stroke each year in the United States, of which 30% are fatal, and hence stroke is the third leading cause of death in the United States. Approximately 80% of strokes are "ischemic" and result from an acute occlusion of a cerebral artery with resultant reduction in blood flow. The remainder are "hemorrhagic", which are due to rupture of a cerebral artery with hemorrhage into brain tissue and consequent obstruction of blood flow due to lack of flow in the distal region of the ruptured vessel and local tissue compression, creating ischemia.

Stroke commonly affects individuals older than 65 years, and the most powerful risk factor is hypertension. Until recently, there was no approved therapy for acute stroke, which was treated by general medical support only, followed by rehabilitation from the observed damage. In 1996, the FDA approved the use of tissue plasminogen activator (tPA) as therapy for acute ischemic stroke, based on a limited number of controlled trials. Some, but not all, of the trials revealed a 30-55% improvement in clinical outcome, with an overall reduction in morbidity, but not mortality. Because of a narrow window of opportunity to treat a patient with tPA and significant side-effects associated with the use of tPA, the drug is underused. Even with thrombolytic therapy with tPA, the therapy produces complete resolution of symptoms less than 40% of the time, so there is need for additional forms of therapy. Numerous neuroprotective strategies have been tested in clinical trials, but none has been approved by the FDA for treating ischemic stroke.

Approximately eighty percent of strokes may be caused by too little blood reaching an area of the brain, which is usually due to a clot that has blocked a blood vessel (*i.e*., for example, a cerebral thrombosis). This is called "ischemic stroke." This type of stroke can sometimes lead to a brain hemorrhage because the affected brain tissue softens and this can lead to breaking down of small blood vessels. In addition, brain hemorrhage can occur when people have problems forming blood clots. Clots, which are the body's way of stopping any bleeding, are formed by proteins called coagulation factors and by sticky blood cells called platelets. Whenever the coagulation factors or platelets do not work well or are insufficient in quantity, people may develop a tendency to bleed excessively.

Ischemic strokes may be preceded by transient ischemic attacks (TIA), and it is estimated that about 300,000 persons suffer a TIA every year in the United States. Thrombosis also contributes to peripheral arterial occlusion in diabetics, sickle cell disease and other patients, and an efficacious and safe anti-ischemic injury agent for use in such patients is needed.

Approximately twenty percent of strokes may involve bleeding within the brain, which damages nearby brain tissue (for example, a hemorrhagic stroke). Hemorrhagic stroke occurs when a blood vessel bursts inside the brain. The brain is very sensitive to bleeding and damage can occur very rapidly, either because of the presence of the blood itself, or because the fluid increases pressure on the brain and harms it by pressing it against the skull. Bleeding irritates the brain tissue, causing swelling. The surrounding tissues of the brain resist the expansion of the bleeding, which is finally contained by forming a mass (for example, an intracerebral hematoma). Both swelling and hematoma will compress and displace normal brain tissue. Most often, hemorrhagic stroke is associated with high blood pressure, which stresses the artery walls until they break.

In Sickle Cell Disease ischemia results from blockage of capillaries and prevention of blood flow into a tissue that becomes starved of oxygen and glucose. This blockade of the capillaries is caused by red blood cells that have lost their normal shape and flexibility, and have collapsed or distorted into the rigid or semi-rigid "sickled" shapes. This blockade of capillaries shuts off the flow of fresh blood through those portions of the organ or tissue that are normally serviced by the blocked capillaries.

For most patients, Sickle Cell Anemia does not cause constant or chronic pain. However, most Sickle Cell Anemia patients suffer from sporadic yet recurrent episodes that are sometimes referred to as ischemic crises. During such crises, a sickle cell patient will usually experience severe pain, at one or more locations which frequently vary between patients, and between different crises in a specific patient. It is not uncommon for one or more joints to become swollen and sore, and/or for the patient to suffer from either sharp or diffuse pain in the abdomen, which is presumed to be due to ischemic conditions in one or more portions of one or more organs.

Most sickle cell patients usually suffer several such ischemic crises per year. During these crises, the patient is usually hospitalized, restricted to bed rest with little or no exertion, and treated with a variety of drugs, including strong painkillers such as morphine, codeine, and meperidine, and by broad-spectrum antibiotics, both to help control any infections that may be contributing to the crises, and to help prevent or reduce additional infections in tissues or organs that are weakened by the ischemic crisis.

Among young children having Sickle Cell Disease, dactylitis is common, due to ischemic necrosis of the small bones and cartilages of the hands and feet, and acute abdominal pain is often caused by accumulating damage to the spleen. Acute abdominal pain can also be due to liver or kidney infarction, or associated with hematuria. Cholecystitis due to gall stones, aseptic necrosis of the head of the femur, radiologic evidence of widened marrow space in the skull, spinal osteoporosis and renal papillary necrosis typically occur over the age of 10 years, and pathological fractures often supervene in patients greater than 18 years, especially at the head of the femur and the humerus. Leg ulcers are common in adult patients, and lobar pneumonias, pulmonary infarctions, stroke, and retinal lesions may occur. In all cases, the pain is severe, often migratory, and diffuse.

One cause of hemorrhagic stroke is an aneurysm. This is a weak spot in an artery wall, which balloons out because of the pressure of the blood circulating inside the affected artery. Eventually, it can burst and cause serious harm. The larger the aneurysm is, the more likely it is to burst.

Stroke symptoms are typically of sudden onset and may quickly become worse. Stroke symptoms may include, but are not limited to: i) Weakness or inability to move a body part; ii) Numbness or loss of sensation; iii) Decreased or lost vision (may be partial); iv) Speech difficulties; v) Inability to recognize or identify familiar things; vi) Sudden headache; vii) Vertigo; viii) Dizziness; xi) Loss of coordination; x) Swallowing difficulties; and xi) Sleepy, stuporous, lethargic, comatose, and/or unconscious.

A stroke event may be detected by using a neurologic exam, which would be expected to show abnormal results. Further, a patient may look drowsy and confused. An eye examination may show abnormal eye movements, and changes may be seen upon retinal examination (examination of the back of the eye with an instrument called ophthalmoscope). The patient may also have abnormal reflexes. A computerized tomography scan will confirm the presence of a brain hemorrhage by providing pictures of the brain. A brain magnetic resonance imaging (MRI) scan can also be obtained later to better understand what caused the bleeding. A conventional angiography (for example, an X-ray of the arteries using dye) may be required to identify aneurysms or AVM. Other tests may include, but are not limited to: complete blood count, bleeding time, prothrombin/partial thromboplastin time (PT/PTT), and CSF (cerebrospinal fluid) examination.

Thrombosis may be defined as the formation, development, or presence of a blood clot (for example, a thrombus) in a blood vessel and is believed to be a common severe medical disorder. Thromboses may be involved in the generation of a variety of vascular disorders including, but not limited to, myocardial infarctions, cardiac ischemia, and/or deep vein thrombosis.

The most frequent example of arterial thrombosis is coronary thrombosis, which leads to occlusion of the coronary arteries and often to myocardial infarction (heart attack). More than 1.3 million patients are admitted to the hospital for myocardial infarction each year in North America. The standard therapy is administration of a thrombolytic protein by infusion. Thrombolytic treatment of acute myocardial infarction is estimated to save 30 lives per 1000 patients treated; nevertheless the 30-day mortality for this disorder remains substantial (Mehta et al., Lancet 356:449-454 (2000). A large part of the tissue damage from thrombosis can be attributed to oxidative damage. It would be convenient to administer antioxidants or agents the inhibit oxidative damage by bolus injection, chronically or acutely.

Unstable angina, caused by inadequate oxygen delivery to the heart due to coronary occlusion, is the most common cause of admission to hospital, with 1.5 million cases a year in the United States alone.

Deep venous thrombosis is a frequent complication of surgical procedures such as hip and knee arthroplasties. Similar considerations apply to venous thrombosis associated with pregnancy and parturition. Some persons are prone to repeated venous thrombotic events and are currently treated by antithrombotic agents such as coumarin-type drugs. The dose of such drugs must be titrated in each patient, and the margin between effective antithrombotic doses and those increasing hemorrhage is small. A combination therapy with an anti-oxidant as part of the therapy would help to reduce oxidative damage associated with thromboli and ischemia.

Deep vein thrombosis may be detected by tests including, but not limited to: i) Doppler ultrasound exam of an extremity blood flow studies; ii) Venography of the legs; or iii) Plethysmography of the legs.

A pulmonary embolus is a blockage of an artery in the lungs by fat, air, blood clot, or tumor cells. Pulmonary emboli are most often caused by blood clots in the veins, especially veins in the legs or in the pelvis (hips). More rarely, air bubbles, fat droplets, amniotic fluid, or clumps of parasites or tumor cells may obstruct the pulmonary vessels. One cause of a pulmonary embolism is a blood clot in the veins of the legs, called a deep vein thrombosis (DVT) (supra). Many clear up on their own, though some may cause severe illness or even death.

Risk factors for a pulmonary embolus may include, but are not limited to: i) Prolonged bed rest or inactivity (including long trips in planes, cars, or trains); ii) Oral contraceptive use; iii) Surgery (especially pelvic surgery); iv) Childbirth; v) Massive trauma; vi) Burns; vii) Cancer; viii) Stroke; ix) Heart attack; x) Heart surgery; or xi) Fractures of the hips or femur. Further, persons with certain clotting disorders and/or autoimmune diseases (*i.e*., for example, anti-cardiolipin antibody syndrome) may also have a higher risk.

Symptoms of pulmonary embolism may be vague, or they may resemble symptoms associated with other diseases. Symptoms can include, but are not limited to: i) Sudden cough; ii) Bloody sputum (significant amounts of visible blood or lightly blood streaked sputum); iii) Sudden onset of shortness of breath at rest or with exertion; iv) splinting of ribs with breathing (bending over or holding the chest); v) chest pain; vi) rapid breathing; or vii) rapid heart rate (tachycardia)

Pulmonary emboli may be identified using tests including, but not limited to: i) Arterial blood gases; ii) Pulse oximetry; iii) Chest x-ray; iv) Pulmonary ventilation/perfusion scan; v) Pulmonary angiogram; vi) electrocardiogram; and v) computerized tomographic chest angiogram.

Thrombophlebitis is swelling (inflammation) of a vein caused by a blood clot. Such conditions are usually a result of sitting for a long period of time (such as on a long airplane trip). Disorders that increase a person's chance for blood clots also lead to thrombophlebitis. Superficial thrombophlebitis affects veins near the skin surface.

Symptoms often associated with superficial thrombophlebitis may include but are not limited to: i) Warmth and tenderness over the vein; ii) Pain in the part of the body affected; iii) Skin redness (not always present); or iv) Inflammation (swelling) in the part of the body affected. Objective tests may be performed to detect thrombophlebitis including, but not limited to: i) Doppler ultrasound; ii) Venography; and iii) Blood coagulation studies.

When an ischemic crisis commences, it sets into motion a cascade of events that render the crisis even more severe and difficult to arrest and treat. Three aspects of the cascading, self-perpetuating nature of these ischemic crises are worth noting. In addition to the reduction of nutrients and oxygen to the site of ischemic injury, the process results in inflammatory damage and production of reactive oxygen species. For example, oxidative stress following ischemic stroke results in the creation of reactive oxygen species such as hydrogen peroxide (H₂O₂), hydroxyl radical (HO•) and superoxide anion radical (O₂•) that cause lipid peroxidation and protein modification leading to neuronal and vascular damage and clinical deficits.

Ischemia also causes tissue damage resulting from the inflammatory cascade leading to leukotriene production via lipoxygenase activation. Lipoxygenases (LOXs) are dioxygenases that incorporate molecular oxygen into polyunsaturated fatty acids, such as arachidonic acid and, based on the site of insertion of the oxygen, are generally classified as 5-, 12-, or 15-LOXs. Recent evidence suggests that 12/15-LOX may play a role in ischemia-induced nerve cell loss. Furthermore, there appears to be a correlation between an early reduction in GSH levels in ischemia and the activation of 12-LOX. Using in *vitro* cell culture assays, 12-LOX inhibitors have been shown to block glutamate-induced cell death and both 5- and 12-LOX inhibitors block ischemic injury in hippocampal slice cultures.

The disclosure, as described more fully below, provides methods and compositions useful for treating any of the foregoing ischemic injuries. In one embodiment, the invention provides use of a cysteamine product (cysteamine, cystamine or a pharmaceutically acceptable salt of any of the foregoing) that reduces ischemic injury directly or indirectly. In one embodiment of the invention, ischemic injury is treated through promoting adiponectin levels in a subject by administration of a cysteamine product.

Adiponectin (sometimes referred to as GBP-28, apMl, AdipoQ and Acrp30) is a protein which in humans is encoded by the ADIPOQ gene. Adiponectin is a protein hormone that modulates a number of metabolic processes, including glucose levels, fatty acid catabolism and inflammation. Adiponectin is secreted from adipose tissue into the bloodstream and is very abundant in plasma relative to many hormones. Levels of the hormone are inversely correlated with body fat in adults. Adiponectin is secreted into the bloodstream where it is about 0.01% of all plasma protein at around 5-10 µg/mL. Levels of adiponectin are reduced in diabetics compared to non-diabetics.

Adiponectin automatically self-associates into larger structures (*e*.*g*., heavy molecular weight (HMW), medium molecular weight (MMW) and low molecular weight (LMW) multimers). Initially, three adiponectin molecules bind together to form a homotrimer. The trimers continue to self-associate and form hexamers or dodecamers. Adiponectin monomers have three domains, an N-terminal signal sequence, a collagenous domain and a C-terminal globular domain. Within the collagenous domain of each LMW trimer an unbound Cys-36 residue can bind to another trimer to form the MMW hexamer, which, in turn can form the larger HMW oligomeric multimer (see, e.g., Figure 2A).

Adiponectin multimers are assembled within the fat cell by a process involving disulfide exchange reactions (probably through hEROs and ERp44) then released to circulate in the blood stream. The release of these proteins into the extracellular space is dependent upon the correct 3-D structural configuration which is achieved through disulfide bond formations (*e*.*g*., through electron transfer and oxidation). Excessive oxidation can lead to aggregation and over-accumulation of the proteins within the cell. Failure to fold through the formation of disulfide bonds will result in the adiponectin proteins remaining intracellularly where they will be degraded. In human cells, Ero1-Lalpha and Ero1-Lbeta (hEROs), continuously oxidize protein disulfide isomerase (PDI)-mediated disulfide bond formation with the help of ER resident proteins, Erp44, which contains a thioredoxin domain. ERp44 forms mixed disulfides (probably via cysteine terminals) with hEROs and cargo folding intermediates. Under normal physiological conditions LMW, MMW, and HMW adiponectin multimers are secreted into the circulation where they are not normally interconvertable. This tightly controlled production and release of adiponectin along with the lack of extracellular inter-conversion between multimers is consistent with adiponectin having different signal-inducing functions in different cells. The LMW form has been shown to be more biologically active than other forms, particularly in promoting insulin action in the liver and skeletal muscle thereby reducing blood sugar levels. Adiponectin has also been demonstrated to play a beneficial role following ischemic injury. In addition, adiponectin has been shown to have a cerebro-protective function through its anti-inflammatory actions. The delivery of adiponectin, however, is difficult and requires additional research and development.

The function of these chaperone proteins is dependent upon optimal intracellular conditions and the mildly oxidizing environment of the endoplasmic reticulum. Excessive oxidation can lead to abnormal folding of the adiponectin molecules, aggregation, intracellular accumulation and ultimately to degradation. Therefore, in conditions associated with oxidative stress such as obesity, diabetes type II, insulin resistance, cardiovascular disease and non-alcoholic fatty liver disease (NAFLD) the production and release of adiponectin will be diminished. Furthermore, subjects having obesity, diabetes, cardiovascular disease and non-alcoholic fatty liver disease are at greater risk for ischemic injury/crises.

As described above, adiponectin biosynthesis is dependent upon the formation and dissolution of mixed disulfide intermediates between adiponectin and endoplasmic reticulum (ER) chaperone proteins. Cysteamine and cystamine are sulfhydryl containing agents, with the capacity to provide reducing equivalents to cellular processes. The disclosure demonstrates that *in vivo* treatment with cysteamine significantly increase circulating levels of adiponectin. Although not wishing to be bound by any particularly mechanism of action, the data suggest that cysteamine may be acting to release disulfide bound, "tethered", pools of cellular adiponectin by donating reducing equivalents. Alternatively, cysteamine may be acting to promote the interconversion of circulating multimeric adiponectin increasing the concentration LMW adiponectin, which possesses the greatest potency of effect at the cellular level. Administration of cysteamine products can further increase the overall amount of adiponectin, including HWM adiponectin. Furthermore, cysteamine has anti-oxidant properties itself, which can stem or reduce the oxidative damage at a site of ischemic injury. Regardless of the multiple mechanisms listed above, the data demonstrate a beneficial effect of cysteamine products i.e., cysteamine or cystamine) in reducing ischemic injury.

Cysteamine (HS-CH₂-CH₂-NH₂) is able to cross cell membranes easily due to its small size. At present, cysteamine is FDA-approved only for the treatment of cystinosis, an intra-lysosomal cystine storage disorder. In cystinosis, cysteamine acts by converting cystine to cysteine and cysteine-cysteamine mixed disulfide which are then both able to leave the lysosome through the cysteine and lysine transporters, respectively (Gahl et al., N Engl J Med, 347(2):111-21, 2002). Treatment with cysteamine has been shown to result in lowering of intracellular cystine levels in circulating leukocytes (Dohil et al., J. Pediatr, 148(6):764-9, 2006). Cysteamine products have not previously been shown to be useful for treating ischemia.

The disclosure provides cysteamine products useful in the treatment of ischemic injury diseases and disorders. Cysteamine products in accordance with the claimed invention are cysteamine, cystamine, or pharmaceutically acceptable salts thereof. "cysteamine product" refers to cysteamine or derivatives thereof, or cystamine or derivatives thereof, a biologically active metabolite thereof, or combination of cysteamine or cystamine, and includes pharmaceutically acceptable salts of any of the foregoing, esters, amides, alkylated compounds, prodrugs, analogs, phosphorylated compounds, sulfated compounds, or other chemically modified forms thereof and the like. Such derivatives include, for example, biologically active metabolites, chemically modified forms of the compound, by such techniques as esterification, alkylation (*e*.*g*., C1, C2 or C3), labeling (*e*.*g*., with radionuclides or various enzymes), covalent polymer attachment such as pegylation (derivatization with polyethylene glycol) or mixtures thereof. In some embodiments, cysteamine products include, but are not limited to, hydrochloride salts, bitartrate salts, phosphorylated derivatives, and sulfated derivatives. Examples of other cysteamine products include 2-aminopropane thiol-1, 1-aminopropane thiol-2, N- and S-substituted cysteamine, AET, aminoalkyl derivatives, phosphorothioate, amifostine (US Patent 4,816,482). In one embodiment, a cysteamine product specifically excludes N-acetylcysteine. In one embodiment, cysteamine products comprise, but are not limited to, structures I and/or II: 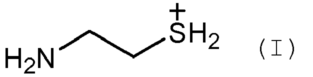 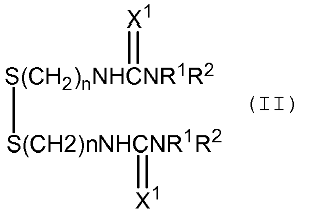 wherein n represents 2 or 3, R₁ and R₂ each represents a hydrogen atom, or an alkyl group optionally substituted by a hydroxy, amino, alkylamino or dialkylamino group, or represents a cycloalkyl or aryl group, and X₁ is selected from the group consisting of =N-CN, =N-NO₂, =N-COR₄, =N-NR-COOR₄, =N-NR-CONH₂, =N-SO₂R₄, =CH-NO₂, -CH-SO₂R₄, =C(CN)₂, =C(CN)COOR₄ and =C(CN)CONH₂, wherein R₄ is an alkyl or aryl group. In another aspect, a cysteamine product can comprise a cysteamine radical linked to any number of non-toxic groups selected from the group consisting of: 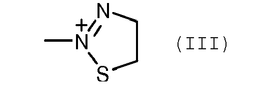 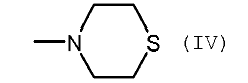 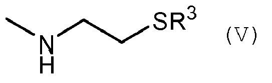 wherein R³ represents hydrogen atom or a straight chain or a branched alkyl group having 1 to 10 carbon atoms.

Pharmaceutically acceptable salts of cysteamine products are also disclosed and comprise pharmaceutically-acceptable anions and/or cations. Pharmaceutically-acceptable cations include among others, alkali metal cations (*e*.*g*., Li⁺, Na⁺, K⁺), alkaline earth metal cations (*e*.*g*., Ca²⁺, Mg²⁺), non-toxic heavy metal cations and ammonium (NH⁴⁺) and substituted ammonium (N(R')⁴⁺, where R' is hydrogen, alkyl, or substituted alkyl, *i*.*e*., including, methyl, ethyl, or hydroxyethyl, specifically, trimethyl ammonium, triethyl ammonium, and triethanol ammonium cations). Pharmaceutically-acceptable anions include among other halides (*e.g*., Cl⁻, Br⁻), sulfate, acetates *(e.g.,* acetate, trifluoroacetate), ascorbates, aspartates, benzoates, citrates, and lactate.

The disclosure is not limited with respect to a specific cysteamine or cystamine salt or ester or derivative; the cysteamine product compositions of the disclosure can contain any cysteamine or cystamine, cysteamine or cystamine derivative, or combination of cysteamine and cystamine. The active agents in the composition, *e.g.,* cysteamine or cystamine, may be administered in the form of a pharmacologically acceptable salt, ester, amide, prodrug or analog or as a combination thereof. Salts, esters, amides, prodrugs and analogs of the active agents may be prepared using standard procedures known to those skilled in the art of synthetic organic chemistry and described, for example, by J. March, "Advanced Organic Chemistry: Reactions, Mechanisms and Structure," 4th Ed. (New York: Wiley-Interscience, 1992). For example, basic addition salts are prepared from the neutral drug using conventional means, involving reaction of one or more of the active agent's free hydroxyl groups with a suitable base. Generally, the neutral form of the drug is dissolved in a polar organic solvent such as methanol or ethanol and the base is added thereto. The resulting salt either precipitates or may be brought out of solution by addition of a less polar solvent. Suitable bases for forming basic addition salts include, but are not limited to, inorganic bases such as sodium hydroxide, potassium hydroxide, ammonium hydroxide, calcium hydroxide, trimethylamine, or the like. Preparation of esters involves functionalization of hydroxyl groups which may be present within the molecular structure of the drug. The esters are typically acyl-substituted derivatives of free alcohol groups, *i.e*., moieties which are derived from carboxylic acids of the formula R-COOH where R is alkyl, and typically is lower alkyl. Esters can be reconverted to the free acids, if desired, by using conventional hydrogenolysis or hydrolysis procedures. Preparation of amides and prodrugs can be carried out in an analogous manner. Other derivatives and analogs of the active agents may be prepared using standard techniques known to those skilled in the art of synthetic organic chemistry, or may be deduced by reference to the pertinent literature.

Cysteamine products can be formulated for targeted delivery or sustained delivery. For example, cysteamine can be enterically coated to promote delivery to the small intestine or at a desired pH of the lower gastrointestinal tract. An enterically coated drug or tablet refers, generally, to a drug or tablet that is coated with a substance (an "enteric coating") that remains intact or substantially intact such that the drug or tablet is passed through the stomach but dissolves and releases the drug in the small intestine. Enteric delivery, delayed release and sustained release formulations are particularly suitable for treating chronic ischemic diseases and disorders such as Sickle Cell Disease.

Any of the formulations of the disclosure can be administered in a sustained release form. The sustained release formulation has the advantage of delivery over an extended period of time without the need for repeated administrations of the formulation.

Sustained release can be achieved, for example, with a sustained release material such as a wafer, an immunobead, a micropump or other material that provides for controlled slow release of the cysteamine product or combination formulation. Such controlled release materials are well known in the art and available from commercial sources. In addition, a bioerodible or biodegradable material can be formulated with active agents of the invention, such as polylactic acid, polygalactic acid, regenerated collagen, multilamellar liposomes or other conventional depot formulations, can be implanted to slowly release the cysteamine product, thrombotic, spin trap, and NMDA antagonist agents. The use of infusion pumps, matrix entrapment systems, and transdermal delivery devices also are contemplated in the invention.

Active agents/formulations also can be advantageously enclosed in micelles or liposomes. Liposome encapsulation technology is well known. Liposomes can be targeted to a specific tissue, such as neural tissue, through the use of receptors, ligands or antibodies capable of binding to an antigen or target in the desired tissue. The preparation of these formulations is well known in the art (see, for example, Pardridge, supra (1991), and Radin and Metz, Meth Enzymol. 98:613-618 (1983)).

The cysteamine product may also include additional pharmaceutically acceptable carriers or vehicles. A pharmaceutically acceptable carrier or vehicle refers, generally, to materials that are suitable for administration to a subject wherein the carrier or vehicle is not biologically harmful, or otherwise, cause undesirable effects. Such carriers or vehicles are typically inert ingredients of a medicament. Typically a carrier or vehicle is administered to a subject along with an active ingredient without causing any undesirable biological effects or interacting in a deleterious manner with any of the other components of a pharmaceutical composition in which it is contained.

A cysteamine product or other active ingredient can comprise a pharmaceutically acceptable salt or ester. For example, salts, esters or other derivatives comprise biologically active forms having a similar biological effect compared to a parent compound. Exemplary salts include hydrochloride salt and bistartrate salts.

Pharmaceutical carriers include pharmaceutically acceptable salts, particularly where a basic or acidic group is present in a compound. For example, when an acidic substituent, such as --COOH, is present, the ammonium, sodium, potassium, calcium and the like salts, are contemplated for administration. Additionally, where an acid group is present, pharmaceutically acceptable esters of the compound (e.g., methyl, tert-butyl, pivaloyloxymethyl, succinyl, and the like) are contemplated as preferred forms of the compounds, such esters being known in the art for modifying solubility and/or hydrolysis characteristics for use as sustained release or prodrug formulations.

When a basic group (such as amino or a basic heteroaryl radical, such as pyridyl) is present, then an acidic salt, such as hydrochloride, hydrobromide, acetate, maleate, pamoate, phosphate, methanesulfonate, p-toluenesulfonate, and the like, is contemplated as a form for administration.

In addition, compounds may form solvates with water or common organic solvents. Such solvates are contemplated as well.

An active ingredient, pharmaceutical or other composition of the disclosure can comprise a stabilizing agent. Stabilizing agents, generally, refer to compounds that lower the rate at which a pharmaceutical degrades, particularly an oral pharmaceutical formulation under environmental conditions of storage. Certain stabilizers are suitable for intravascular delivery. For example, one or more of the following stabilizers can be used in formulating a cysteamine product for intravascular delivery: α-tocopherol, 2,6-di-tert-butyl-4-methylphenol (BHT), tocopherol acetate, 2-tert-butyl-4-hydroxyanisole and/or 3-tert-butyl-4-hydroxyanisole (BHA), dodecyl gallate, acetate and ascorbic acid.

As used herein, a "therapeutically effective amount" or "effective amount" refers to that amount of cysteamine, cystamine, or salts thereof sufficient to result in amelioration of symptoms, for example, treatment, healing, prevention or amelioration of the relevant medical condition, or an increase in rate of treatment, healing, prevention or amelioration of such conditions, typically providing a statistically significant improvement in the treated population. When referencing an individual active ingredient, administered alone, a therapeutically effective dose refers to that ingredient alone. When referring to a combination, a therapeutically effective dose refers to combined amounts of the active ingredients that result in the therapeutic effect, whether administered in combination, including serially or simultaneously. In one embodiment, a therapeutically effective amount of the cysteamine product ameliorates symptoms, including but not limited to, inflammatory damage, oxidative damage, reduction in the amount of oxidative agents, reduction in ischemic injury resulting from an acute or chronic ischemic event, an increase in adiponectin levels and/or increase of LMW adiponectin.

As used herein, the term "an ischemic injury alleviating amount" or "effective amount" means the amount of a composition comprising a cysteamine product useful for causing a diminution in ischemic injury, whether by alleviating tissue damage, alleviating behavioral changes, or by promoting reperfusion of the damaged tissue. An effective amount to be administered systemically depends on the body weight of the subject. Typically, an effective amount to be administered systemically is about 0.1 mg/kg to about 100 mg/kg (e.g., about 0.5 mg/kg to about 20 mg/kg). An effective amount will of course depend upon a number of factors including, for example, the age and weight of the subject (e.g., a mammal such as a human), the precise condition requiring treatment and its severity, the route of administration, and will ultimately be at the discretion of the attendant physician or veterinarian.

Pharmaceutical compositions of the disclosure containing a cysteamine product as an active ingredient may contain pharmaceutically acceptable carriers or additives depending on the route of administration. Examples of such carriers or additives include water, a pharmaceutically acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, glycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the like. Additives used are chosen from, but not limited to, the above or combinations thereof, as appropriate, depending on the dosage form of the disclosure.

Formulation of the pharmaceutical composition will vary according to the route of administration selected (e.g., solution, emulsion, coated tablet). An appropriate composition comprising the cysteamine product to be administered can be prepared in a physiologically acceptable vehicle or carrier. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. Intravenous vehicles can include various additives, preservatives, or fluid, nutrient or electrolyte replenishers.

A variety of aqueous carriers, e.g., water, buffered water, 0.4% saline, 0.3% glycine, or aqueous suspensions may contain the active compound in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

In some embodiments, the cysteamine product of this disclosure can be lyophilized for storage and reconstituted in a suitable carrier prior to use. Any suitable lyophilization and reconstitution techniques can be employed. It is appreciated by those skilled in the art that lyophilization and reconstitution can lead to varying degrees of activity loss and that use levels may have to be adjusted to compensate.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

In one embodiment, the disclosure provides use of an enterically coated cysteamine product composition. Enteric coatings prolong release until the cysteamine product reaches the intestinal tract, typically the small intestine. Because of the enteric coatings, delivery to the small intestine is improved thereby improving uptake of the active ingredient while reducing gastric side effects.

In some embodiments, the coating material is selected such that the therapeutically active agent is released when the dosage form reaches the small intestine or a region in which the pH is greater than pH 4.5. The coating may be a pH-sensitive material, which remain intact in the lower pH environment of the stomach, but which disintegrate or dissolve at the pH commonly found in the small intestine of the patient. For example, the enteric coating material begins to dissolve in an aqueous solution at pH between about 4.5 to about 5.5. For example, pH-sensitive materials will not undergo significant dissolution until the dosage form has emptied from the stomach. The pH of the small intestine gradually increases from about 4.5 to about 6.5 in the duodenal bulb to about 7.2 in the distal portions of the small intestine. In order to provide predictable dissolution corresponding to the small intestine transit time of about 3 hours (*e*.*g*., 2-3 hours) and permit reproducible release therein, the coating should begin to dissolve at the pH range within the small intestine. Therefore, the amount of enteric polymer coating should be sufficient to substantially dissolved during the approximate three hour transit time within the small intestine, such as the proximal and midintestine.

Enteric coatings have been used for many years to arrest the release of the drug from orally ingestible dosage forms. Depending upon the composition and/or thickness, the enteric coatings are resistant to stomach acid for required periods of time before they begin to disintegrate and permit release of the drug in the lower stomach or upper part of the small intestines. Examples of some enteric coatings are disclosed in U.S. Pat. No. 5,225,202 which is incorporated by reference fully herein. As set forth in U.S. Pat. No. 5,225,202, some examples of coating previously employed are beeswax and glyceryl monostearate; beeswax, shellac and cellulose; and cetyl alcohol, mastic and shellac, as well as shellac and stearic acid (U.S. Pat. No. 2,809,918); polyvinyl acetate and ethyl cellulose (U.S. Pat. No. 3,835,221); and neutral copolymer of polymethacrylic acid esters (Eudragit® L30D) (F. W. Goodhart et al., Pharm. Tech., pp. 64-71, April 1984); copolymers of methacrylic acid and methacrylic acid methylester (Eudragits), or a neutral copolymer of polymethacrylic acid esters containing metallic stearates (Mehta et al., U.S. Pat. Nos. 4,728,512 and 4,794,001). Such coatings comprise mixtures of fats and fatty acids, shellac and shellac derivatives and the cellulose acid phthlates, *e*.*g*., those having a free carboxyl content. See, Remington's at page 1590, and Zeitova et al. (U.S. Pat. No. 4,432,966), for descriptions of suitable enteric coating compositions. Accordingly, increased adsorption in the small intestine due to enteric coatings of cysteamine product compositions can result in improved efficacy.

Generally, the enteric coating comprises a polymeric material that prevents cysteamine product release in the low pH environment of the stomach but that ionizes at a slightly higher pH, typically a pH of 4 or 5, and thus dissolves sufficiently in the small intestines to gradually release the active agent therein. Accordingly, among the most effective enteric coating materials are polyacids having a pKa in the range of about 3 to 5. Suitable enteric coating materials include, but are not limited to, polymerized gelatin, shellac, methacrylic acid copolymer type C NF, cellulose butyrate phthalate, cellulose hydrogen phthalate, cellulose proprionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and acrylic acid polymers and copolymers, typically formed from methyl acrylate, ethyl acrylate, methyl methacrylate and/or ethyl methacrylate with copolymers of acrylic and methacrylic acid esters (Eudragit® NE, Eudragit® RL, Eudragit® RS). For example, the enterically coating can comprise Eudragit® L30D, triethylcitrate, and hydroxypropylmethylcellulose (HPMC), wherein the coating comprises 10 to 13% of the final product.

In one embodiment, the cysteamine product composition is administered in tablet form. In various embodiments, tablets are manufactured by first enterically coating the cysteamine product. A method for forming tablets herein is by direct compression of the powders containing the enterically coated cysteamine product, optionally in combination with diluents, binders, lubricants, disintegrants, colorants, stabilizers or the like. In various embodiments, the tablets are manufactured by blending excipients with the cysteamine product, compressing the tablet and enteric coating. As an alternative to direct compression, compressed tablets can be prepared using wet-granulation, dry- granulation or roller compaction processes. Tablets may also be molded rather than compressed, starting with a moist material containing a suitable water-soluble lubricant. Biologically available iron may be included in an gastric/oral formulation to promote uptake of the cyteamine product.

The disclosure provides a method of treating ischemic injury and inflammation associated with oxidative damage comprising administering a cysteamine product, e.g., cysteamine or derivative thereof, or a cystamine or derivative thereof, in an amount effective to reduce oxidative damage in a subject at a site of ischemic injury. The methods and compositions include delivering a cysteamine product to a subject prior to, simultaneously with or subsequent to an ischemic event. In some or any embodiments, the cysteamine product is delivered simultaneously with or within a time period immediately after, an acute ischemic event, in an amount effective to reduce ischemic injury. The administered cysteamine products have one or more effects including reducing oxidative damage, promoting adiponectin production/circulation to inhibit oxidative damage.

The disclosure provides methods and compositions for promoting adiponectin levels in a subject comprising administering to a subject an effective amount of a cysteamine product, *e*.*g*., cysteamine or a derivative thereof, or cystamine or a derivative thereof, or pharmaceutically acceptable salt thereof. The disclosure demonstrates that contacting a cell or subject with a cysteamine product increases the level of adiponectin, compared to the same cell or subject in the absence of the cysteamine product. In one embodiment, the cysteamine or derivative thereof, or cystamine or a derivative thereof, or pharmaceutically acceptable salt thereof can promote the production of a low molecular weight multimer of adiponectin.

The disclosure provides a method of treating ischemic injury and inflammation comprising administering a cysteamine product, *e*.*g*., cysteamine or derivative thereof, or a cystamine or derivative thereof, or pharmaceutically acceptable salt thereof, in an amount effective to increase adiponectin levels and/or modulate GSH levels in a subject. The methods and compositions include delivering a cysteamine product to a subject prior to, simultaneously with or subsequent to an ischemic event. In some or any embodiments, the cysteamine product is delivered simultaneously with or within a time period immediately after, an acute ischemic event, in an amount effective to reduce ischemic injury and/or increase adiponectin levels. In one embodiment, the increased adiponectin levels are increased levels of a low molecular weight adiponectin multimer.

The disclosure provides methods and compositions for increasing adiponectin levels and particularly LMW adiponectin. Furthermore, the disclosure provides methods and compositions to treat ischemic injury. In other embodiment, the disclosure provides treating a subject in need of increased adiponectin levels (*e*.*g*., LMW adiponectin) with a cysteamine product in an amount effective to increase the levels of adiponectin. The subject may be a subject having an acute ischemic event, ischemic injury or likely to have an acute ischemic event. The subject may be a subject having a thrombotic disorder. The subject may be an obese subject. In some embodiments, the obese subject has low LMW adiponectin levels, or high HMW adiponectin levels, compared to a population of healthy subjects (*e*.*g*., non-obese subjects). According to the claimed invention, the subject is not a diabetic subject and does not have hypercholesterolemia.

The disclosure provides methods and compositions to treat chronic diseases that increase the risk of ischemia. Chronic diseases that have an increased risk of ischemic injury/crises include, but are not limited to, Atherosclerosis (lipid-laden plaques obstructing the lumen of arteries); hypoglycemia (lower than normal level of glucose); tachycardia (abnormally rapid beating of the heart); hypotension (low blood pressure, *e.g.* in septic shock, heart failure); thromboembolism (blood clots; *e.g.,* associated with cancer); outside compression of a blood vessel, *e.g.* by a tumor or in the case of superior mesenteric artery syndrome; embolism (foreign bodies in the circulation, *e.g.* amniotic fluid embolism); Sickle Cell Disease (abnormally shaped red blood cells); and anemia. In treating such chronic disease compositions that are dosed less frequently, can be taken orally vs. IV and sustained/delayed release formulations are particularly useful. For example, the disclosure provides enterically coated cysteamine products that are release at a pH higher than 4.5 and that are dosed less frequently than Cystagon® are provided. The formulation for such delivery may also include a bioavailable iron supplement or component to promote uptake of the cysteamine product. Where the subject is in a clinical setting, IV administration can be used over extended periods of time. For example, a cysteamine product can be formulated in a pharmaceutically acceptable buffer comprising stabilizers for IV administration.

The disclosure also provides a method for inhibiting damage to jeopardized tissue during reperfusion in a mammal, which comprises administering to the mammal an effective amount of a cysteamine product alone or in combination with other therapeutic agents (*e.g.,* a thrombolytic, free-radical scavenger and the like) as described herein.

The agents may be administered simultaneously or sequentially in separate formulations or may be administered simultaneously in a single formulation. In any event the delay in administering the second or third etc. of a plurality of agents should not be such as to lose the benefit of a potentiated effect of the combination of the agents *in vivo* in inhibiting tissue damage.

As described above, the cysteamine product compositions may be prepared for administration orally, parenterally, transocularly, intranasally, transdermally, transmucosally, by inhalation spray, vaginally, rectally, into the cerebral spinal fluid, or by intracranial injection. The term parenteral as used herein includes subcutaneous injections, intravenous, intramuscular, intracisternal injection, or infusion techniques. Administration by intravenous, intradermal, intramusclar, intramammary, intraperitoneal, intrathecal, retrobulbar, intrapulmonary injection and or surgical implantation at a particular site is contemplated as well. Generally, compositions for administration by any of the above methods are essentially free of pyrogens, as well as other impurities that could be harmful to the recipient. Further, compositions for administration parenterally are sterile.

Direct intracranial injection or injection into the cerebrospinal fluid also can be used to introduce an effective amount of a cysteamine product. The cysteamine product may be combined with other therapeutic agents as described herein.

Intravascular infusions are normally carried out with the parenteral solution contained within an infusion bag or bottle or within an electrically operated infusion syringe. The solution may be delivered from the infusion bag or bottle to the subject by gravity feed or by the use of an infusion pump. The use of gravity feed infusion systems in some instances does not afford sufficient control over the rate of administration of the parenteral solution and, therefore, the use of an infusion pump may be desirable especially with solutions containing relatively high concentrations of spin trap/thrombolytic formulation. An electrically operated infusion syringe may offer even greater control over the rate of administration.

For treatment of neural damage associated with, for example, acute ischemic stroke, an appropriate daily systemic dosage of a cysteamine product formulation (*e*.*g*., comprising cysteamine, or cystamine of either of the foregoing) is based on the body weight of the subject and is in the range of from about 0.1 pg/kg to about 100 mg/kg, although dosages from about 0.1 mg/kg to about 100 mg/kg, or from about 0.5 mg/kg to about 20 mg/kg are also contemplated. Thus, for the typical 70 kg human, a systemic dosage can be between about 7 µg and about 7,000 mg daily. A daily dosage of locally administered material will be about an order of magnitude less than the systemic dosage. Oral administration is also contemplated.

The cysteamine product can be administered locally to a site of ischemic injury (*e*.*g*., acute ischemic injury) or may be administered systemically. In one embodiment, the cysteamine product may be administered chronically or continuously over a period of time to a subject that may be at risk for ischemic injury (*e*.*g*., subject having Sickle Cell disease). The route and formulation for administration may differ depending upon chronic or acute treatments.

In one embodiment, the cysteamine product can be administered acutely at the time of ischemic injury or within a time period immediately after the ischemic event or ischemic injury. The time period may range from a few minutes (e.g., 1, 2, 3, 4, 5, or more minutes) or 10, 20, 30, 60, 90 120 or more minutes to several hours after an ischemic event (*e*.*g*., 3 hours, 6, hours, 10 hours, 12 hours or more). In one embodiment, the cysteamine product may be administered in combination with one or more additional agents useful for treating ischemia. For example, the method may include administration with a reperfusion agent, a free-radical scavenger agent, a spin trap agent or the like.

In another embodiment, the subject is at risk for stroke, cardiac ischemia or other ischemic injury, *e*.*g*., has experienced or is experiencing conditions that create a risk for stroke, pulmonary or cardiac ischemia. Examples of such conditions include high blood pressure; tobacco use; diabetes mellitus; carotid or other artery disease; peripheral artery disease; atrial fibrillation; other heart disease; transient ischemic attacks (TIAs); certain blood disorders (*e*.*g*., high red blood cell count; Sickle cell disease); high blood cholesterol; physical inactivity and obesity; excessive alcohol; some illegal drugs; a prior stroke; or prior heart attack.

In chronic conditions (*e*.*g*., Sickle Cell Disease), sustained or controlled release formulations are applicable. In acute conditions (*e*.*g*., ischemic stroke) an immediate release formulation is appropriate such as through IV delivery (or other parenteral delivery), local cerebral delivery and the like. In some embodiments, the cysteamine product composition is a delayed or controlled release dosage form that provides a Cₘₐₓ of the cysteamine product that is at least about 35%, 40%, 45%, 50%, 55%, 60%, 65%, 70%, 75%, 80%, 85%, or 100% higher than the Cₘₐₓ provided by an immediate release dosage form containing the same amount of the cysteamine product. In some embodiments, the Cₘₐₓ is up to about 75%, 100%, 125% or 150% higher than the Cₘₐₓ of the immediate release dosage form. Cₘₐₓ refers to the maximum dose of the cysteamine product in the blood after dosing and provides an indicator that the drug is absorbed systemically.

In some embodiments, the AUC of the delayed or controlled release dosage form is also increased by at least about 20%, 25%, 30%, 35%, 40%, 45%, or 50%, or up to about 50%, 60%, 75% or 100% relative to an immediate release dosage form. AUC or "area under the curve", and refers to the kinetic curve derived when plasma drug concentration versus time is measured after dosing of a drug.

The preparation of delayed, controlled or sustained/extended release forms of pharmaceutical compositions with the desired pharmacokinetic characteristics is known in the art and can be accomplished by a variety of methods. For example, oral controlled delivery systems include dissolution-controlled release (*e*.*g*., encapsulation dissolution control or matrix dissolution control), diffusion-controlled release (reservoir devices or matrix devices), ion exchange resins, osmotic controlled release or gastroretentive systems. Dissolution controlled release can be obtained, *e*.*g*., by slowing the dissolution rate of a drug in the gastrointestinal tract, incorporating the drug in an insoluble polymer, and coating drug particles or granules with polymeric materials of varying thickness. Diffusion controlled release can be obtained, *e*.*g*., by controlling diffusion through a polymeric membrane or a polymeric matrix. Osmotically controlled release can be obtained, *e.g.,* by controlling solvent influx across a semipermeable membrane, which in turn carries the drug outside through a laser-drilled orifice. The osmotic and hydrostatic pressure differences on either side of the membrane govern fluid transport. Prolonged gastric retention may be achieved by, *e.g.,* altering density of the formulations, bioadhesion to the stomach lining, or increasing floating time in the stomach. For further detail, see the Handbook of Pharmaceutical Controlled Release Technology, Wise, ed., Marcel Dekker, Inc., New York, NY (2000), incorporated by reference herein in its entirety, *e.g.* Chapter 22 ("An Overview of Controlled Release Systems").

The concentration of cysteamine product in these formulations can vary widely, for example from less than about 0.5%, usually at or at least about 1% to as much as 15 or 20% by weight and are selected primarily based on fluid volumes, manufacturing characteristics, viscosities, etc., in accordance with the particular mode of administration selected. Actual methods for preparing administrable compositions are known or apparent to those skilled in the art and are described in more detail in, for example, Remington's Pharmaceutical Science, 15th ed., Mack Publishing Company, Easton, Pa. (1980).

The cysteamine product is present in the composition in a therapeutically effective amount; typically, the composition is in unit dosage form. The amount of cysteamine product administered will, of course, be dependent on the age, weight, and general condition of the subject, the severity of the condition being treated, and the judgment of the prescribing - physician. Suitable therapeutic amounts will be known to those skilled in the art and/or are described in the pertinent reference texts and literature. Current non-enterically coated doses are about 1.35 g/m² body surface area and are administered 4-5 times per day. In one embodiment, the dose is administered either one time per day or multiple times per day for chronic disease states (*e.g.,* Sickle Cell Disease) and can be administered in a bolus or IV for acute conditions (*e.g.,* acute ischemic injury). The cysteamine product may be administered one, two or three or four times per day. In certain embodiments, the cysteamine product is given less than four times per day. In some embodiments, an effective dosage of cysteamine product may be within the range of 0.01 mg to 1000 mg per kg (mg/kg) of body weight per day, or from about 0.1 mg/kg to about 100 mg/kg, or from about 0.5 mg/kg to about 20 mg/kg of body weight per day. Further, the effective dose may be 0.5 mg/kg, 1 mg/kg, 5 mg/kg, 10 mg/kg, 15 mg/kg, 20 mg/kg/ 25 mg/kg, 30 mg/kg, 35 mg/kg, 40 mg/kg, 45 mg/kg, 50 mg/kg, 55 mg/kg, 60 mg/kg, 70 mg/kg, 75 mg/kg, 80 mg/kg, 90 mg/kg, 100 mg/kg, 125 mg/kg, 150 mg/kg, 175 mg/kg, 200 mg/kg, and may increase by 25 mg/kg increments up to 1000 mg/kg, or may range between any two of the foregoing values. In some embodiments, the cysteamine product is administered at a total daily dose of from approximately 0.25 g/m² to 4.0 g/m² body surface area, e.g., at least about 0.5, 0.6, 0.7, 0.8, 0.9, 1.0, 1.1, 1.2, 1.3, 1.4, 1.5, 1.6, 1.7, 1.8, 1.9 or 2 g/m², or up to about 0.8, 0.9, 1.0, 1.1, 1.2, 1.3, 1.4, 1.5, 1.6, 1.7, 1.8, 1.9, 2.0, 2.2, 2.5, 2.7, 3.0, or 3.5 g/m². In some embodiments, the cysteamine product may be administered at a total daily dose of about 0.5 to 2.0 g/m² body surface area, 1-1.5 g/m² body surface area, or 0.5-1 g/m² body surface area, or about 0.7-0.8 g/m² body surface area, or about 1.35 g/m² body surface area. Salts or esters of the same active ingredient may vary in molecular weight depending on the type and weight of the salt or ester moiety. For administration of the dosage form, e.g., a tablet or capsule or other oral dosage form comprising the enterically coated cysteamine product, a total weight in the range of approximately 100 mg to 1000 mg is used. The dosage form is orally administered to a patient suffering from, for example, (i) a disease associated with irregular or abnormal total adiponectin levels, (ii) irregular or abnormal ratios of HMW:MMW, HMW:LMW, or MMW:LMW adiponectin, (iii) chronic ischemic risk or events, or (iv) acute ischemic injury (*e*.*g*., stroke or other cardiovascular disease). Administration may continue for several hours, days, weeks, months or years depending upon the disease or disorder (*e*.*g*., acute event or chronic disease).

Compositions useful for administration may be formulated with uptake or absorption enhancers to increase their efficacy. Such enhancers include, for example, salicylate, glycocholate/linoleate, glycholate, aprotinin, bacitracin, SDS, caprate, iron and the like. See, *e*.*g*., Fix (J. Pharm. Sci., 85:1282-1285, 1996) and Oliyai and Stella (Ann. Rev. Pharmacol. Toxicol., 32:521-544, 1993).

The enterically coated cysteamine product can comprise various excipients, as is well known in the pharmaceutical art, provided such excipients do not exhibit a destabilizing effect on any components in the composition. Thus, excipients such as binders, bulking agents, diluents, disintegrants, lubricants, fillers, carriers, and the like can be combined with the cysteamine product. For solid compositions, diluents are typically necessary to increase the bulk of a tablet so that a practical size is provided for compression. Suitable diluents include dicalcium phosphate, calcium sulfate, lactose, cellulose, kaolin, mannitol, sodium chloride, dry starch and powdered sugar. Binders are used to impart cohesive qualities to a tablet formulation, and thus ensure that a tablet remains intact after compression. Suitable binder materials include, but are not limited to, starch (including corn starch and pregelatinized starch), gelatin, sugars (including sucrose, glucose, dextrose and lactose), polyethylene glycol, waxes, and natural and synthetic gums, *e*.*g*., acacia sodium alginate, polyvinylpyrrolidone, cellulosic polymers (including hydroxypropyl cellulose, hydroxypropyl methylcellulose, methyl cellulose, hydroxyethyl cellulose, and the like), and Veegum. Lubricants are used to facilitate tablet manufacture; examples of suitable lubricants include, for example, magnesium stearate, calcium stearate, and stearic acid, and are typically present at no more than approximately 1 weight percent relative to tablet weight. Disintegrants are used to facilitate tablet disintegration or "breakup" after administration, and are generally starches, clays, celluloses, algins, gums or crosslinked polymers. If desired, the pharmaceutical composition to be administered may also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, and the like. If desired, flavoring, coloring and/or sweetening agents may be added as well. Other optional components for incorporation into an oral formulation herein include, but are not limited to, preservatives, suspending agents, thickening agents, and the like. Fillers include, for example, insoluble materials such as silicon dioxide, titanium oxide, alumina, talc, kaolin, powdered cellulose, microcrystalline cellulose, and the like, as well as soluble materials such as mannitol, urea, sucrose, lactose, dextrose, sodium chloride, sorbitol, and the like.

A pharmaceutical composition may also comprise a stabilizing agent such as hydroxypropyl methylcellulose or polyvinylpyrrolidone, as disclosed in U.S. Pat. No. 4,301,146. Other stabilizing agents include, but are not limited to, cellulosic polymers such as hydroxypropyl cellulose, hydroxyethyl cellulose, methyl cellulose, ethyl cellulose, cellulose acetate, cellulose acetate phthalate, cellulose acetate trimellitate, hydroxypropyl methylcellulose phthalate, microcrystalline cellulose and carboxymethylcellulose sodium; and vinyl polymers and copolymers such as polyvinyl acetate, polyvinylacetate phthalate, vinylacetate crotonic acid copolymer, and ethylene-vinyl acetate copolymers. The stabilizing agent is present in an amount effective to provide the desired stabilizing effect; generally, this means that the ratio of cysteamine product to the stabilizing agent is at least about 1:500 w/w, more commonly about 1:99 w/w.

The tablets can be manufactured by first enterically coating the cysteamine product. In one embodiment, a method for forming tablets herein is by direct compression of the powders containing the enterically coated cysteamine product, optionally in combination with diluents, binders, lubricants, disintegrants, colorants, stabilizers or the like. In various embodiments, the tablets are manufactured by blending excipients with the cysteamine product, compressing the tablet and enteric coating. As an alternative to direct compression, compressed tablets can be prepared using wet-granulation, dry-granulation or roller compaction processes. Tablets may also be molded rather than compressed, starting with a moist material containing a suitable water-soluble lubricant.

In an alternative embodiment, the enterically coated cysteamine products are granulated and the granulation is compressed into a tablet or filled into a capsule. Capsule materials may be either hard or soft, and are typically sealed, such as with gelatin bands or the like. Tablets and capsules for oral use will generally include one or more commonly used excipients as discussed herein. In various embodiments, the capsule is a non-sealing capsule.

For administration of the dosage form, *i.e*., the tablet or capsule comprising the enterically coated cysteamine product, a total weight in the range of approximately 100 mg to 1000 mg is used. The dosage form is orally administered to a patient suffering from a condition for which a cysteamine product would typically be indicated, including, but not limited to, ischemic diseases and disorders (*e*.*g*., sickle cell disease, acute ischemic events, stroke, heart attack and the like).

The cysteamine product compositions of the disclosure can be used in combination with other therapies useful for treating ischemic events or disease and disorder associated with abnormal adiponectin levels or ratios. For example, the cysteamine may be administered intravenously in combination with a thrombolytic agent, a free-radical scavenger, a spin trap agent and the like.

Combining two or more drugs with differing mechanisms of action can provide maximal ischemic injury protection via additive or synergistic effects or provide additional benefit by increasing the therapeutic window for compounds or possibly by reducing side-effects. While there is a need to reduce the consequences of activation of the ischemic cascade, thrombolytics are valuable agents because they produce recanalization. This allows not only for reperfusion of ischemic tissue when the stroke is the result of a thrombus or embolus, but also provides improved access of small molecules, including drugs and nutrients to the penumbra of the infarcted tissue.

The methods and compositions of the disclosure can include, in addition to the cysteamine product, (1) an antioxidant; (2) a thrombolytic agent, (3) an NMDA receptor antagonist and (4) a spin trap agent; or (5) any combination of the foregoing.

Antioxidants include, but are not limited to, nitrones (STAZN), polyphenols, flavonols (*e*.*g*., baicalein) and phenylpropanoids (chlorogenic acid and fisetin), are useful in the methods and compositions of the disclosure as neuroprotection and to treat ischemic injury resulting from stroke. In various embodiments, the antioxidant combined with cysteamine product thereof excludes N-acetylcysteine.

Various thrombolytic agents are also known in the art (*e*.*g*., Tenecteplase) and/or in combination with a spin trap agent (*e*.*g*., NXY-059) are useful in the methods or compositions of the invention. Any number of thrombolytic agents can be used in the methods and compositions of the invention. Examples of thrombolytic agents that can be used in the methods and composition of the invention include alteplase, tenecteplase, reteplase, streptase, abbokinase, pamiteplase, nateplase, desmoteplase, duteplase, monteplase, reteplase, lanoteplase, and Prolyse™). Other thrombolytics include, for example, microplasmin, Bat-tPA, BB-10153 (an engineered form of human plasminogen activated to plasmin by thrombin) and Desmodus rotundus salivary plasminogen activators (DSPAs) (*e.g*., DSPAα1).

Phenolic acids include, for example, caffeic acid, vanillin, and courmaric acid. Phenolic acids form a diverse group that includes the widely distributed hydroxybenzoic and hydroxycinnamic acids. Hydroxycinnamic acid compounds (p-coumaric, caffeic acid, ferulic acid) occur most frequently as simple esters with hydroxy carboxylic acids or glucose, while the hydroxybenzoic acid compounds (p-hydroxybenzoic, gallic acid, ellagic acid) are present mainly in the form of glucosides. Often phenolic acids occur as esters or glycosides conjugated with flavonoids, alcohols, hydroxyfatty acids, sterols, and glucosides.

Nitrone-based spin trap agents such as NXY-059 and thrombolytics, such as Tenecteplase, are currently being developed for the treatment of acute ischemic stroke (AIS)-since they are two of the most promising drug candidates.

Spin traps such as nitroxides and nitrones are stabilized forms of the biological messenger nitric oxide. Unlike other antioxidants, spin traps neither act as proxidants, nor do they propagate free radical chain reactions. Likewise, these agents inhibit the reaction of superoxide and nitric oxide to produce peroxinitrite. Thus, combination therapies with spin traps and therapeutic agents currently under development or in use for such diseases and disorders as Parkinsonism, stroke, ischaemic injury, heart attack, and age-related dementias are encompassed by the invention.

Nitrone and nitroso spin trap compounds are commercially available. Exemplary nitrone and nitroso spin trap compounds include disodium 2,4-disulfophenyl-*N*-tert-butylnitrone (NXY-059), N-t-butyl-α-phenylnitrone, 3,5-dibromo-4-nitrosobenzenesulfonic acid, 5,5-dimethyl-1-pyrroline N-oxide, 2-methyl-2-nitrosopropane, nitrosodisulfonic acid, α-(4-pyridyl-1-oxide)-N-t-butylnitrone, 3,3,5,5-tetramethylpyrroline N-oxide, 2,4,6-tri-t-butylnitrosobenzene, PTIYO (4-phenyl-2,2,5,5-tetramethyl imidazolin-1-yloxy-5-oxide) and tempol (4-hydroxy 2,2,6,6-tetramethylpiperidine-1-oxyl) and the like.

In another aspect of the invention, methods and formulations comprising an NMDA (N-Methyl-D-Aspartate) receptor antagonists are provided that, in combination with a thrombolytic agent, improve behavioral performance. Examples of NMDA receptor antagonists include 3-alpha-ol-5-beta-pregnan-20-one hemisuccinate, ketamine, memantine, dextromethorphan, dextrorphan, and dextromethorphan hydrobromide. Piperidine derivatives and analogues substituted with phenols or phenol equivalents having NR2B selective NMDA antagonist activity are described in international patent application nos. WO 90/14087, WO 90/14088, WO 97/23202, WO 97/23214, WO 97/23215, WO 97/23216, WO 97/23458, WO 99/21539, WO 00/25109, European patent application No. EP 648744 A1 and in U.S. Pat. No. 5,436,255. Compounds containing 2-benzoxazolinone substructure with the same biological activity are described in international patent applications WO 98/18793 and WO 00/00197. Other NR2B selective NMDA antagonists having condensed heterocyclic structures are described in international patent application nos. WO 01/30330, WO 01/32171, WO 01/32174, WO 01/32177, WO 01/32179, 01/32615, WO 01/32634.

Assays for determining the efficacy of a particular therapy comprising a cysteamine product alone or in combination with one or more additional agents can be performed in an embolic stroke model, which produces a behavioral endpoint to monitor and allows comparison of the efficacy of compound(s). Not only can behavioral endpoints be monitored but also histochemical techniques can be used to measure endpoints and pathology.

The effectiveness of a method or composition of the disclosure can also be assessed by measuring adiponectin levels and/or ratios of the various adiponectin multimers. In addition, the effectiveness can be assessed by clinical observations, diagnostics and the like that are indicative of tissue damage resulting from ischemic injury. Dosage adjustment and therapy can be made by a medical specialist depending upon, for example, the severity of an ischemic event or a disease or disorder associated with aberrant or abnormal adiponectin levels.

Several ischemic injury animal models can be used to assess the efficacy of a treatment of the disclosure. For example, endothelin-1 is a potent vasoconstrictor which is produced endogenously during ischemic stroke and which contributes to overall loss of cells and disability. Exogenous endothelin-1 can also be used to induce stroke and cell death after sustained vasoconstriction with reperfusion. It can be microinjected to induce focal stroke in small tissue volumes (*e*.*g*., cortical grey matter, white matter or subcortical tissue) or after injection near the middle cerebral artery. It is often used as a model of focal stroke to evaluate candidate pro-regenerative therapies. One advantage of this model of stroke is that it causes highly reproducible infarcts with very low mortality.

In another model, middle cerebral artery (MCA) occlusion is achieved in this model by injecting particles like blood clots (thromboembolic MCAO) or artificial spheres into the carotid artery of animals as an animal model of ischemic stroke. Thromboembolic MCAO is achieved either by injecting clots that were formed *in vitro* or by endovascular instillation of thrombin for *in situ* clotting. The thromboembolic model is closest to the pathophysiology of human cardio embolic stroke. When injecting spheres into the cerebral circulation, their size determines the pattern of brain infarction: Macrospheres (300 - 400 pm) induce infarcts similar to those achieved by occlusion of the proximal MCA, whereas microsphere (∼ 50 pm) injection results in distal, diffuse embolism. However, the quality of MCAO - and thus the volume of brain infarcts - is very variable, a fact which is further aggravated by a certain rate of spontaneous lysis of injected blood clots.

In another animal model of ischemic stroke the MCA is surgically dissected and subsequently permanently occluded, e.g., by electrocautery or ligation. Occlusion can be performed on the proximal or distal part of the MCA. In the latter, ischemic damage is restricted to the cerebral cortex. MCAO can be combined with temporal or permanent common carotid artery occlusion. These models require a small craniotomy.

A transient transcranial MCAO model can be used and is similar to that of permanent transcranial MCAO, with the MCA being reperfused after a defined period of focal cerebral ischemia. Like permanent MCAO, craniotomy is required and common carotid artery (CCA) occlusion can be combined. Occluding one MCA and both CCAs is referred to as the three vessel occlusion model of focal cerebral ischemia.

The cysteamine products can be assayed for their use in any of the foregoing animal models by administering the therapeutic agent before, during or after inducement of an ischemic injury.
The effectiveness of the therapy can be measured by histology or behavioral deficits compared to a control which did not receive cysteamine product.

In yet other embodiments, the methods of the disclosure can be assayed by isolating adipocytes and incubating the adipocytes with or without a cysteamine product. The cysteamine product may be contact with the adipocytes at various dosages. The effectiveness can be assayed by measuring a change in adiponectin levels or multimers in cell culture, wherein an increase in adiponectin or a change in ratio of adiponectin multimers is indicative of an agent having an effect on adiponectin levels.

The following Examples are meant to further illustrate the invention, but not limit the foregoing disclosure or the appended claims.

### EXAMPLES

Cysteamine bitartrate (Mylan Pharma,VA) capsules were enterically-coated by The Coating Place Inc. (Verona, WI) using a Model 600 Wurster unit.

### EXAMPLE 1

Fat biopsy adipose tissue (AT) samples were collagenase digested x 1hr at 37°C in a rotating waterbath. Digested AT was then filtered through 425pm nylon mesh to separate fat cells. Isolated fat cells (FC) were twice washed in physiologic salts buffer (Phillips 2008), filtered and twice washed again. FC were spun at 50xg to concentrate. 1 ml FC were resuspended in 9 ml of defined culture media (Phillips 2008) and FC were allowed to recover x 10 hrs overnight at 37°C.

Adipocytes were isolated and allowed to recover x overnight then cultured with or without 90 µM cysteamine and media collected at the following time points (45, 90, 180, 24 hr). Western Blot analysis of 20 µl of media was performed with non-reducing 5X Loading buffer. Blotted, blocked and incubated with primary antibody (Anti-adiponectin mouse monoclonal at 1:500 overnight). The blot was washed and incubated with a secondary antibody (Goat anti-mouse IR 800) and densitometry measured on a LICOR densitometer. (see Figure 1).

To determine the time course of cysteamine effects on adiponectin, media was changed and FC were resuspended in 9 ml of defined media ± 90 µM cysteamine. FC content and secretion of adiponectin into the media was assessed in duplicate culture at 0, 45, 90, 180 minutes and 24 hr. For analysis of FC content, isolated FCs were extracted as previously described (Phillips, 2008). Proteins were separated by non-reducing, non-heat denaturing SDS PAGE electophoresis and transferred to nitrocellulose for immunobloting using 1:500 primary monoclonal anti-adiponectin antibody (BD Transduction Laboratories) and 1:20,000 LiCOR Odyssey secondary antibody. Signal detection and quantitative analysis was completed using the LiCOR Odyssey Machine (LiCOR, Lincoln, NE). Multimeric content of cellular adiponectin was determined by dividing the AU for the multimer by the total AU per sample. Multimeric content of conditioned media was determined as for cell content then this percentage was multiplied by total adiponectin in the media as determined by ELISA (Millipore Billerica, Massachusetts).

### EXAMPLE 2

Eleven children ≥10 years, with biopsy-proven NAFLD and an alanine aminotransferase (ALT) level ≥60 IU/L, were enrolled. They were either newly diagnosed or had not received specific therapy for NAFLD such as vitamin E or insulin-sensitizing drugs for at least 3 months before entering the study. All had undertaken standard of care lifestyle changes involving diet and exercise for more than 3 months. The study's primary outcome measure was normalization (≤40 IU/L) or ≥50% reduction in baseline serum ALT at 24 weeks of EC-cysteamine therapy; these subjects were deemed responders.

Subjects were treated for 24wks and remained monitored for 24 weeks following its discontinuation. The mean EC-cysteamine dose ingested was 654mg twice daily or 15.2 mg/kg /day (median dose 900mg) for 24wks.

During the pilot study blood was collected at pre-defined intervals. Serum was stored at -80°C until time of assay. Adequate serum volumes were available in 10 of the 11 subjects with NAFLD and only up to 16 weeks post-treatment.

Total and multimeric adiponectin levels were measured in serum taken from: (1) subject with NAFLD collected at 0 and 24wks of cysteamine therapy and again 16wks after stopping therapy (*i.e*., 40 weeks); (2) pretreated 0-week subjects with NAFLD and adults without NAFLD at room temperature and at 37 °C and also after incubation with cysteamine 90 µM for 1 hour at 37°C; and (3) pretreated 0-week subjects with NAFLD before and after incubation with cysteamine at varying concentrations (15, 30, 45, 60, 90, 120 µM) for 1 hour at 37 °C to establish dose-response profile.

Sodium dodecyl sulfate polyacrylamide gel electrophoresis (SDS-PAGE) was performed according to standard Laemmli's method. Sample buffer for non-reducing, non-heat denaturing conditions contained 3% SDS; 50 mmol/l Tris-HCL, pH 6.8 and 20% glycerol. Proteins were separated using 3-8% Tris-Acetate Gels (Invitrogen, Carlsbad CA) with Tris-Acetate running buffer per manufacturer instructions. For immunoblotting, proteins were transferred to nitrocellulose membranes. Membranes were washed with Tween (Tris-buffered Saline, 0.1% Triton-X-100) and incubated with monoclonal antibody (1:500 BD Transduction Laboratories, San Diego, CA). After washing with Tris-buffered saline with 0.1% Tween (4 x 5 min) membranes were incubated with L-COR secondary antibodies (LI-COR Biotechnology, Lincoln NE) at 1:20,000. Adiponectin multimers were detected and quantified using the LICOR imager and software analysis program. Relative distribution of adiponectin multimers was determined by dividing band density by total density (cellular adiponectin) or by total adiponectin as determined by ELISA (Millipore, Billerica, MA). The percentage of adionectin multimer was then multiplied by the total adiponectin level to calculate absolute oligomer value.

In another experiment, 25µl total sample volume (5µl diluted serum + 15µl water + 5µl 5X LB) was incubated with or without cysteamine at 37°C x 1hr. A control was prepared without cysteamine and incubated at room temperature for 1 hour on the bench. The preparations were then run on 2 x 15-well 1.5mm 3-8% Tris-acetate gels. Primary antibody - mouse anti-adiponectin 1:500, overnight at 4°C and secondary antibody - goat anti-mouse IR800 1:20,000.

Serum was analyzed from 10 subjects at baseline and 24 weeks of treatment with cysteamine and again at 16 weeks after therapy was discontinued (*i.e*., 40 weeks after enrollment). Seven of the 10 subjects were responders. Three subjects had a reduction in AST/ALT levels but did not reach the study's endpoint. Compared with baseline, there was no significant change in mean BMI measurements at 24 weeks (P = .47) or at 40 weeks (P = .43). For all study subjects, there was a reduction in mean ALT from baseline (123 IU/L) at 24 weeks (55 IU/L, P = .003) and at 40 weeks (71 IU/L, P = .055). There was a reduction in mean AST from baseline (60.9 IU/L) at 24 weeks (32 IU/L, P = .001), and at 40 weeks (35 IU/L, P = .012). For the responders, the mean leptin levels were 21.3, 15.7, and 19.5 ng/mL, respectively, with a significant reduction between 0- and 24- week timepoints (P = .04) (Figure 2B).

**Healthy Subjects -1 Hour 90 µM Cysteamine.** HMW levels were unchanged compared with baseline. Similar to subjects with NAFLD, a mean percent increase in LMW levels (P < .005) and also a mean percent reduction in MMW levels (P < .01) was detected compared with baseline (Figure 7). The mean percentage of total adiponectin level that comprised HMW, MMW, LMW multimers changed from 42.9%, 46.0%, and 11.1%, respectively, to 42.7%, 39.3%, and 18.0%, respectively, following incubation with cysteamine.

To determine threshold dose of cysteamine on reduction of serum adiponectin multimers, serum was collected and incubated with various concentrations of cysteamine. Six microliters of diluted GB serum was incubated with 24 µl water and various concentrations of cysteamine. Samples, were incubated in a tissue culture incubator for 1hr at 37 °C with a control tube having no cysteamine addition. To each tube was then added 5 µl of 5 X non-reducing loading buffer. The samples were vortexed and loaded on a 3-8% tris-Acetate gel and run for 75 min at 125 V constant current. The gel was transferred and blocked. The blot was then incubated overnight with anti-adiponectin antibody 1:500 in 3% BSA/TBS. The blot was washed and incubated with a secondary anti-mouse antibody for 1 hour. Densitometry was performed on a LICOR densitometer. The results were quantitated and are presented in Figures 8-9.

**Adiponectin Multimers-24 weeks EC-cysteamine.** For all subjects, following 24 weeks of cysteamine bitartrate therapy, the mean percent increase for HMW, MMW, LMW, and total adiponectin from baseline was 53% (P = .02), 19% (P = .02), 29.4% (P = .03), and 49.3% (P = .05), respectively (Figure 3 and 5). Between the end of treatment at the 24-week and at the 40-week timepoints, there was a significant mean percent reduction in HMW (P = .02), MMW (P = .01), LMW (P = .009), and total adiponectin (P = .01). At the 40-week timepoint, all adiponectin multimer levels were within 2% of baseline. This did not follow the trend for mean ALT and AST levels, which did not return to baseline levels at 40 weeks. The total adiponectin levels increased from 0 week by 49.3% at 24 weeks (P = .05) and decreased from 24 weeks to baseline levels at 40 weeks (P = .01). Although levels of all multimer forms increased following 24 weeks of cysteamine therapy, the relative proportions of adiponectin multimer increased significantly only for HMW (12.2%-14.9%, P = .02), decreased for MMW (73.7%-70.3%, P = .03), and remained unchanged for LMW (14.1%-14.8%). For the 7 subjects with NAFLD who were responders the mean percent change for HMW, MMW, LMW, and total adiponectin at 24 weeks increased from baseline by 87.7%, 13.7%, 33.9%, and 57.5%, respectively.

**Adiponectin Multimers-1 hour cysteamine.** No differences were noted between pretreatment adiponectin multimer levels at room temperature and 37°C (Figure 4). Incubation with cysteamine for 1 hour at 37°C resulted in a mean percent increase in LMW levels (P < .0001) and also a mean percent reduction in MMW levels (P < .0001) compared with pretreatment. HMW levels were unchanged compared with baseline. The mean percentage of total adiponectin level that comprised of HMW, MMW, and LMW multimers changed from 8.2%, 63.3%, and 28.5%, respectively, to 8.6%, 54.7%, and 36.7%, respectively, following cysteamine incubation. See Figure 13.

**Dose Response Profile.** HMW levels increased by about 10% above baseline when serum was incubated in cysteamine at concentrations of 15, 30, 45, and 60 µM. MMWlevels were lower than baseline at all cysteamine concentrations. Mean LMW levels increased by >60% above baseline following treatment with cysteamine at all concentrations used (Figure 11). The greatest percent increase in LMW, 77%, was achieved at 120 µM cysteamine concentration, and this coincided with reductions in HMW and MMW levels below baseline.

### EXAMPLE 3

The data above shows that cysteamine has two effects on adiponectin multimerization, (1) an *in vivo* effect following daily treatment in humans will increase all adiponectin multimer levels, and (2) an *in vitro* effect with rapid and significant elevation in LMW adiponectin in human serum exposed to 90µM cysteamine for 1 hr. To date there are only a limited number of drugs that increase circulating adiponectin levels, but none that alter the multimeric distribution of adiponectin.

A previous study in adiponectin knockout mice that underwent ischemia/reperfusion studies showed that those animals that received recombinant adiponectin had less myocardial ischemic damage on histology and also retained more of their myocardial function. Recombinant adiponectin is not readily available. The data above shows that cysteamine has rapidly increased serum LMW adiponectin levels in serum taken from humans with NAFLD and also in healthy adult controls. In addition, cysteamine is known to be a potent antioxidant and has also been shown to induce glutathione, scavenge reactive oxygen species, inhibit tissue transglutaminase activity as well as radio-protective properties.

To analyze ischemic damage and treatment with cysteamine a study in mice (C57BL/6-Male-12 weeks, 25g) that are either pre-treated or naive to cysteamine bitartrate was performed as follows.

Pre-treated animals received intra-peritoneal cysteamine, 60mg/kg twice (120mg/kg/day) 2 hours before surgery for LAD artery ligation. Following a 60 minute ligation period the obstruction was removed and another dose of cysteamine was given immediately at the time of reperfusion and also twice daily injections for 4 days following reperfusion. A control group received the same volumes of control IV solution injected instead of cysteamine. Acceptable volumes of blood were collected at baseline, and at predetermined time points after the ischemic insult in order to determine levels of surrogate markers of inflammation and plasma cysteamine. Animals underwent echocardiography/hemodynamic studies immediately after IR, and 3 days post IR. Before sacrifice, the LAD was re-occluded and Evan's Blue was injected through JVC in order to delineate the non-ischemic area. Myocardial tissue was sent for 2,3,5 triphenyltetrazolium chloride (differentiates between metabolically active and inactive or infracted tissue), H&E and also TUNEL stain. Echocardiography provided ventricular volume data as well as wall function. Hemodynamic studies provided muscle contractility data (specifically systolic data).

So far seven mice were studied. Four received control solution and 3 received cysteamine. Control mice had a mean initial weight of 25.8g (range 25-27g) and a terminal weight of 22.5g (21-24g). This is a reduction of 13%. Animals were lethargic post-thoracotomy and did not feed well.

Cysteamine mice had a mean initial weight of 21.3g (range 21-22g) and a terminal weight of 18.3g (18-19g). This is a reduction 14%. Animals were lethargic post-thoracotomy and did not feed well.

Animals did receive regular subcutaneous saline boluses after the surgery, but, may have been partly dehydrated at the time of the hemodynamic/Echo studies. However, both groups were similarly affected. Dehydration may impact hemodynamic/Echo studies by reducing volume and function.

**Ischemic damage.** Between the cysteamine and control groups there was a significant reduction in infarct area (IA) to area at risk (AAR) (*i*.*e*., IA/AAR) (p=0.004) and IA/LV (left ventricle) (p=0.008) ratios. The relative amounts of ischemic damage were about half in cysteamine treated compared with control groups, (see, *e*.*g*., Fig. 14A-B; raw data in Tables A-B).

**Table A:**

| | LV AREA | AAR | IA | AAR/LV (control) (%) | IA/AAR (control) (%) | IA/LV (control) (%) |
|---|---|---|---|---|---|---|
| RD21 | 110.80 | 27.32 | 18.61 | 24.65 | 68.13 | 16.80 |
| RD22 | S5.61 | 25.75 | 15.32 | 30.08 | 59.48 | 17.89 |
| RD24 | 90.99 | 27.27 | 14.19 | 29.97 | 52.05 | 15.60 |
| RD26 | 79.94 | 29.71 | 16.23 | 37.26 | 54.61 | 20.35 |
| **MEAN** | **91.84** | **27.51** | **16.09** | **30.49%** | **58.57%** | **17.66%** |
| **SD** | **13.42** | **1.636** | **1.877** | **5.176** | **7.081** | **2.023** |

| | | | | | | |
|---|---|---|---|---|---|---|
| **Raw data for 4 control mice receiving acetate/ascorbic acid.** LV = Left Ventricle AAR = Area at Risk IA = Ischemic Area | | | | | | |

**Table B:**

| | LV AREA | AAR | IA | AAR/LV (cysteamine) (%) | IA/AAR (cysteamine) (%) | IA/LV (cysteamine) (%) |
|---|---|---|---|---|---|---|
| RD27 | 73.99 | 24.64 | 9.37 | 33.31 | 38.02 | 12.66 |
| RD28 | 178.34 | 32.02 | 11.14 | 17.95 | 34.78 | 6.24 |
| RD29 | 63.25 | 12.84 | 3.46 | 20.31 | 26.92 | 5.47 |
| **MEAN** | **105.2** | **23.17** | **7.99** | **23.86%** | **33.24%** | **8.12%** |
| **SD** | **63.57** | **9.675** | **4.022** | **8.271** | **5.708** | **3.948** |

| | | | | | | |
|---|---|---|---|---|---|---|
| **Raw data for 3 mice receiving cysteamine mixed with acetate/ascorbic acid.** LV = Left Ventricle AAR = Area at Risk IA = Ischemic Area | | | | | | |

The percentage IA/AAR and IA/LV was about half in the cysteamine teated group compared with the control group.

**End diastolic volume (EDV).** Increase in EDV after MI would suggest ventricular dilatation due to ischemic damage. An interval reduction (between 24 and 72h) would be desirable. There was no significant difference between mean EDV measured at 24h and 72h in the control group (p=0.59). However, there was a significant difference between mean EDV measured at 24h and 72h in the cysteamine group (p=0.032) (see, *e*.*g*., Fig. 15A-B).

**EDV percentage change.** To compare EDV in the two groups the percentage change in actual ventricular volume (pL) was calculated from the 24h time point for each of the two groups. The cysteamine treated group had a significantly greater percentage reduction in EDV (compared with 24h) at the72h time point compared with the control group (p=0.04) (see Fig. 16).

**End systolic volume (ESV)**. An increase in ESV after MI would suggest reduced ventricular contractility. There was a significant difference between mean ESV measured at 24h and 72h in the control (p=0.01) and cysteamine (p=0.025) groups. (see, *e*.*g*., 17A-B) .

**ESV percentage change.** To compare ESV in the two groups the percentage change in actual ventricular volume from the 24h time point for each of the two groups was compared. The cysteamine treated group had a significantly greater percentage reduction in ESV at the 72h time point compared with the control group (p=0.039) (see, *e*.*g*., Fig. 18).

## Claims

1. A cysteamine, cystamine, or a pharmaceutically acceptable salt of any of the foregoing for use in treating ischemia, ischemic injury, an acute ischemic event, thrombosis or an ischaemic injury resulting from thrombosis in a subject, wherein the use comprises increasing adiponectin levels in the subject
and wherein the subject does not have diabetes or hypercholesterolemia.

2. A cysteamine, cystamine, or a pharmaceutically acceptable salt of any of the foregoing for use according to claim 1, wherein the ischemic injury is the result of a thrombotic disorder preferably selected from sickle cell disease, deep vein thrombosis, pulmonary embolism, cardiac embolism, hypercoagulable state, thrombophilia, Factor V Leiden, Antithrombin III deficiency, Protein C deficiency, Protein S deficiency, Prothrombin gene mutation (G20210A), Hyperhomocysteinemia, antiphospholipid antibody syndrome (APS), anticardiolipin antibody (ACLA) thrombosis syndrome, and lupus anticoagulant (LA) syndrome,
and /or wherein cysteamine, cystamine, or the pharmaceutically acceptable salt of any of the forgoing is cysteamine or a pharmaceutically acceptable salt thereof.

3. A cysteamine, cystamine, or a pharmaceutically acceptable salt thereof for use according to claim 1 or claim 2, wherein the the cysteamine, cystamine, or the pharmaceutically acceptable salt of any of the foregoing is in an amount effective to reduce the risk of ischemic injury resulting from thrombosis.

4. A cysteamine, cystamine, or a pharmaceutically acceptable salt of any of the foregoing for use according to claim 2 or claim 3, wherein the subject has cancer, myeloproliferative disorder, multiple myeloma, surgery, trauma, immobilization, oral contraceptive use, administration of thalidomide or its congeners optionally in combination with steroid, heparin-induced thrombocytopenia, pregnancy, inflammatory bowel disease, nephrotic syndrome, paroxysmal nocturnal hemoglobinuria, hyperviscosity syndrome, or Waldenstrom's macroglobulinemia.

5. A cysteamine, cystamine, or a pharmaceutically acceptable salt of any of the foregoing for use according to any preceding claim, wherein the ischemic injury is the result of a tissue injury, a disease or a stroke, or wherein the injury is a reperfusion injury, preferably wherein the reperfusion injury is a result of surgery or organ transplant, or wherein the disease is Sickle Cell Disease.

6. A cysteamine, cystamine, or a pharmaceutically acceptable salt of any of the foregoing for use according to any preceding claim, wherein the cysteamine, cystamine, or the pharmaceutically acceptable salt of any of the foregoing is administered prior to, simultaneously with or after an ischemic event/crisis.

7. A cysteamine, cystamine, or a pharmaceutically acceptable salt of any of the foregoing for use according to any preceding claim, wherein the cysteamine, cystamine, or the pharmaceutically acceptable salt of any of the foregoing is formulated for oral administration, and optionally,
wherein the cysteamine, cystamine, or a pharmaceutically acceptable salt of any of the foregoing is enterically coated, wherein preferably, the enteric coating is a coating selected from polymerized gelatin, shellac, methacrylic acid copolymer type C NF, cellulose butyrate phthalate, cellulose hydrogen phthalate, cellulose propionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and acrylic acid polymers and copolymers, typically formed from methyl acrylate, ethyl acrylate, methyl methacrylate and/or ethyl methacrylate with copolymers of acrylic and methacrylic acid esters.

8. A cysteamine, cystamine, or a pharmaceutically acceptable salt of any of the foregoing for use according to any of claims 1 to 6, wherein the cysteamine, cystamine, or a pharmaceutically acceptable salt of any of the foregoing is formulated for intraperitoneal administration or is administered intravascularly.

9. A cysteamine, cystamine, or a pharmaceutically acceptable salt of any of the foregoing for use according to any preceding claim, wherein the use comprises increasing the production of a low molecular weight (LMW) multimer of adiponectin.

10. A cysteamine, cystamine, or a pharmaceutically acceptable salt of any of the foregoing for use according to any preceding claim, wherein the use comprises increasing the ratio of lower molecular weight (LMW) adiponectin to medium and/or high molecular weight adiponectin in the subject, optionally wherein the subject is obese and has low LMW adiponectin levels compared to healthy subjects.

11. A cysteamine, cystamine, or a pharmaceutically acceptable salt thereof for use according to any preceding claim wherein the subject has a risk factor for ischemia selected from high blood pressure, tobacco use, carotid or other artery disease, peripheral artery disease, atrial fibrillation, other heart disease, transient ischemic attacks (TIAs), a blood disorder, physical inactivity, obesity, excessive alcohol use, illegal drug use, a prior stroke, Sickle Cell Anemia and prior heart attack,
and/or wherein the subject has previously suffered from an ischemic event.

12. A cysteamine, cystamine, or a pharmaceutically acceptable salt of any of the foregoing for use according to any preceding claim, wherein the cysteamine, cystamine, or the pharmaceutically acceptable salt of any of the foregoing is administered at a concentration in the range of 0.5-20 mg/kg of body weight of said subject,
and/or wherein the cysteamine, cystamine, or the pharmaceutically acceptable salt of any of the foregoing, is administered in a total daily dose of 0.5-4.0 g/m².

13. A cysteamine, cystamine, or a pharmaceutically acceptable salt of any of the foregoing for use according to any preceding claim, wherein the cysteamine, cystamine, or the pharmaceutically acceptable salt of any of the foregoing is formulated for administration with one or more additional agents useful to treat ischemia, preferably wherein the one or more additional agents is selected from a reperfusion agent, a free-radical scavenger agent, and a spin trap agent, a neuroprotective agent, an anticoagulant, an antiplatelet agent, nimodipine, and naloxone,
and/or wherein the cysteamine, cystamine, or the pharmaceutically acceptable salt of any of the foregoing is formulated for administration less than four times per day, preferably for administration twice daily,
and/or wherein the cysteamine, cystamine, or the pharmaceutically acceptable salt of any of the foregoing is formulated for administration continuously for a period of days, weeks or months.

14. A cysteamine, cystamine, or a pharmaceutically acceptable salt of any of the foregoing for use according to any preceding claim, formulated for administration with one or more further agents useful to treat a thrombotic disorder, optionally an anticoagulant, heparin, a vitamin K antagonist, 4-hydroxycoumarin derivatives, warfarin, acenocoumarol, dicumarol, ethyl biscoumacetate, phenprocoumon, streptokinase, urokinase, tissue plasminogen activator (tPA), alteplase (recombinant tPA), reteplase, tenecteplase, and argatroban.

15. A cysteamine, cystamine, or a pharmaceutically acceptable salt of any of the foregoing for use according to any preceding claim, wherein cysteamine, cystamine, or the pharmaceutically acceptable salt of any of the foregoing is administered orally with a bioavailable iron formulation, and/or wherein the use comprises a reduction in inflammation from the ischemic event.

## Patentansprüche

1. Cysteamin, Cystamin oder pharmazeutisch akzeptables Salz eines beliebigen der Vorstehenden zur Verwendung bei der Behandlung von Ischämie, ischämischer Verletzung, einem akuten ischämischen Ereignis, Thrombose oder einer ischämischen Verletzung, die aus Thrombose resultiert, bei einem Probanden, wobei die Verwendung ein Erhöhen von Adiponektinspiegeln in dem Probanden umfasst und wobei der Proband keinen Diabetes oder keine Hypercholesterinämie hat.

2. Cysteamin, Cystamin oder pharmazeutisch akzeptables Salz eines beliebigen der Vorstehenden zur Verwendung nach Anspruch 1, wobei die ischämische Verletzung das Resultat einer Thromboseerkrankung ist, die vorzugsweise aus Sichelzellerkrankung, tiefer Venenthrombose, Lungenembolie, kardialer Embolie, Hyperkoagulabilitätszustand, Thrombophilie, Faktor-V-Leiden, Antithrombin-III-Mangel, Protein-C-Mangel, Protein-S-Mangel, Prothrombin-Genmutation (G20210A), Hyperhomocysteinämie, Antiphospholipid-Antikörper-Syndrom (APS), Anticardiolipin-Antikörper-Thrombose-Syndrom (ACLA-Thrombose-Syndrom) und Lupusantikoagulans-Syndrom (LA-Syndrom) ausgewählt ist,
und/oder wobei es sich Cysteamin, Cystamin oder dem pharmazeutisch akzeptablen Salz eines beliebigen der Vorstehenden um Cysteamin oder ein pharmazeutisch akzeptables Salz davon handelt.

3. Cysteamin, Cystamin oder pharmazeutisch akzeptables Salz eines beliebigen der Vorstehenden zur Verwendung nach Anspruch 1 oder 2, wobei das Cysteamin, Cystamin oder das pharmazeutisch akzeptable Salz eines beliebigen der Vorstehenden in einer Menge ist, die dazu wirksam ist, das Risiko einer ischämischen Verletzung, die aus Thrombose resultiert, zu senken.

4. Cysteamin, Cystamin oder pharmazeutisch akzeptables Salz eines beliebigen der Vorstehenden zur Verwendung nach Anspruch 2 oder 3, wobei der Proband Krebs, eine myeloproliferative Erkrankung, ein multiples Myelom, einen chirurgischen Eingriff, ein Trauma, eine Immobilisation, eine Verwendung eines oralen Kontrazeptivums, eine Verabreichung von Thalidomid oder seiner Kongenere, gegebenenfalls in Kombination mit einem Steroid, eine Heparin-induzierte Thrombozytopenie, eine Schwangerschaft, eine entzündliche Darmerkrankung, ein nephrotisches Syndrom, eine paroxysmale nächtliche Hämoglobinurie, ein Hyperviskositätssyndrom oder einen Morbus Waldenström hat.

5. Cysteamin, Cystamin oder pharmazeutisch akzeptables Salz eines beliebigen der Vorstehenden zur Verwendung nach einem vorhergehenden Anspruch, wobei die ischämische Verletzung das Resultat einer Gewebeverletzung, einer Krankheit oder eines Schlaganfalls ist oder wobei die Verletzung eine Reperfusionsverletzung ist, vorzugsweise wobei die Reperfusionsverletzung ein Resultat eines chirurgischen Eingriffs oder einer Organtransplantation ist, oder wobei es sich bei der Krankheit um eine Sichelzellerkrankung handelt.

6. Cysteamin, Cystamin oder pharmazeutisch akzeptables Salz eines beliebigen der Vorstehenden zur Verwendung nach einem vorhergehenden Anspruch, wobei das Cysteamin, Cystamin oder das pharmazeutisch akzeptable Salz eines beliebigen der Vorstehenden vor, gleichzeitig mit oder nach einem ischämischen Ereignis/einer ischämischen Krise verabreicht wird.

7. Cysteamin, Cystamin oder pharmazeutisch akzeptables Salz eines beliebigen der Vorstehenden zur Verwendung nach einem vorhergehenden Anspruch, wobei das Cysteamin, Cystamin oder das pharmazeutisch akzeptable Salz eines beliebigen der Vorstehenden zur oralen Verabreichung formuliert ist und gegebenenfalls
wobei das Cysteamin, Cystamin oder ein pharmazeutisch akzeptables Salz eines beliebigen der Vorstehenden enterisch beschichtet ist, wobei vorzugsweise die enterische Beschichtung eine Beschichtung ist, die aus polymerisierter Gelatine, Schellack, Methacrylsäure-Copolymer-Typ C NF, Cellulosebutyratphthalat, Cellulosehydrogenphthalat, Cellulosepropionatphthalat, Polyvinylacetatphthalat (PVAP), Celluloseacetatphthalat (CAP), Celluloseacetattrimellitat (CAT), Hydroxypropylmethylcellulosephthalat, Hydroxypropylmethylcelluloseacetat, Dioxypropylmethylcellulosesuccinat, Carboxymethylethylcellulose (CMEC), Hydroxypropylmethylcelluloseacetatsuccinat (HPMCAS) und Acrylsäure-Polymeren und -Copolymeren, in der Regel aus Methylacrylat, Ethylacrylat, Methylmethacrylat und/oder Ethylmethacrylat mit Copolymeren von Acryl- und Methacrylsäureestern gebildet, ausgewählt ist.

8. Cysteamin, Cystamin oder pharmazeutisch akzeptables Salz eines beliebigen der Vorstehenden zur Verwendung nach einem der Ansprüche 1 bis 6, wobei das Cysteamin, Cystamin oder ein pharmazeutisch akzeptables Salz eines beliebigen der Vorstehenden zur intraperitonealen Verabreichung formuliert ist oder intravaskulär verabreicht wird.

9. Cysteamin, Cystamin oder pharmazeutisch akzeptables Salz eines beliebigen der Vorstehenden zur Verwendung nach einem vorhergehenden Anspruch, wobei die Verwendung ein Erhöhen der Produktion eines niedermolekularen (NM) Multimers von Adiponektin umfasst.

10. Cysteamin, Cystamin oder pharmazeutisch akzeptables Salz eines beliebigen der Vorstehenden zur Verwendung nach einem vorhergehenden Anspruch, wobei die Verwendung ein Erhöhen des Verhältnisses von niedermolekularem (NM) Adiponektin zu mittel- und/oder hochmolekularem Adiponektin in dem Probanden umfasst, gegebenenfalls wobei der Proband adipös ist und im Vergleich zu gesunden Probanden niedrige NM-Adiponektin-Spiegel aufweist.

11. Cysteamin, Cystamin oder pharmazeutisch akzeptables Salz eines beliebigen der Vorstehenden zur Verwendung nach einem vorhergehenden Anspruch, wobei der Proband einen Risikofaktor für Ischämie hat, der aus hohem Blutdruck, Tabakkonsum, einer Krankheit der Halsschlagader oder einer anderen Arterie, einer peripheren arteriellen Krankheit, Vorhofflimmern, einer anderen Herzkrankheit, transienten ischämischen Attacken (TIA), eine Bluterkrankung, körperlicher Inaktivität, Adipositas, übermäßigem Alkoholkonsum, Verwendung illegaler Drogen, einem früheren Schlaganfall, Sichelzellanämie und einem früheren Herzanfall ausgewählt ist,
und/oder wobei der Proband zuvor ein ischämisches Ereignis erlitten hat.

12. Cysteamin, Cystamin oder pharmazeutisch akzeptables Salz eines beliebigen der Vorstehenden zur Verwendung nach einem vorhergehenden Anspruch, wobei das Cysteamin, Cystamin oder das pharmazeutisch akzeptable Salz eines beliebigen der Vorstehenden in einer Konzentration im Bereich von 0,5-20 mg/kg Körpergewicht des Probanden verabreicht wird
und/oder wobei das Cysteamin, Cystamin oder das pharmazeutisch akzeptable Salz eines beliebigen der Vorstehenden in einer Gesamttagesdosis von 0,5-4,0 g/m² verabreicht wird.

13. Cysteamin, Cystamin oder pharmazeutisch akzeptables Salz eines beliebigen der Vorstehenden zur Verwendung nach einem vorhergehenden Anspruch, wobei das Cysteamin, Cystamin oder das pharmazeutisch akzeptable Salz eines beliebigen der Vorstehenden zur Verabreichung mit einem oder mehreren zusätzlichen Mittel, die zur Behandlung von Ischämie von Nutzen sind, formuliert ist, vorzugsweise wobei das eine oder die mehreren zusätzlichen Mittel aus einem Reperfusionsmittel, einem Radikalfängermittel und einem Spin-Trap-Mittel, einem Neuroprotektivum, einem Antikoagulans, einem Antithrombozytenmittel, Nimodipin und Naloxon ausgewählt sind,
und/oder wobei das Cysteamin, Cystamin oder das pharmazeutisch akzeptable Salz eines beliebigen der Vorstehenden zur kontinuierlichen Verabreichung für einen Zeitraum von Tagen, Wochen oder Monaten formuliert ist.

14. Cysteamin, Cystamin oder pharmazeutisch akzeptables Salz eines beliebigen der Vorstehenden zur Verwendung nach einem vorhergehenden Anspruch, das zur Verabreichung mit einem oder mehreren weiteren Mitteln, die zur Behandlung einer Thromboseerkrankung von Nutzen sind, formuliert ist, gegebenenfalls einem Antikoagulans, Heparin, einem Vitamin-K-Antagonisten, 4-Hydroxycumarinderivaten, Warfarin, Acenocumarol, Dicumarol, Ethylbiscumacetat, Phenprocumon, Streptokinase, Urokinase, Gewebeplasminogenaktivator (tPA), Alteplase (rekombinanter tPA), Reteplase, Tenecteplase und Argatroban.

15. Cysteamin, Cystamin oder pharmazeutisch akzeptables Salz eines beliebigen der Vorstehenden zur Verwendung nach einem vorhergehenden Anspruch, wobei Cysteamin, Cystamin oder das pharmazeutisch akzeptable Salz eines beliebigen der Vorstehenden oral mit einer biologisch verfügbaren Eisenformulierung verabreicht wird und/oder wobei die Verwendung eine Verringerung einer Entzündung von dem ischämischen Ereignis umfasst.

## Revendications

1. Cystéamine, cystamine ou sel pharmaceutiquement acceptable de l'une quelconque de celles-ci pour une utilisation dans le traitement d'une ischémie, d'une lésion ischémique, d'un événement ischémique aigu, d'une thrombose ou d'une lésion ischémique résultant d'une thrombose chez un sujet, où l'utilisation comprend une augmentation des taux d'adiponectine chez le sujet et où le sujet ne présente pas un diabète ou une hypercholestérolémie.

2. Cystéamine, cystamine ou sel pharmaceutiquement acceptable de l'une quelconque de celles-ci pour une utilisation selon la revendication 1, où la lésion ischémique est le résultat d'une maladie thrombotique de préférence sélectionnée parmi les suivantes :
drépanocytose, thrombose veineuse profonde, embolie pulmonaire, embolie cardiaque, hypercoagulabilité, thrombophilie, déficience en facteur V Leiden, déficience en antithrombine III, déficience en protéine C, déficience en protéine S, mutation du gène de la prothrombine (G20210A), hyperhomocystéinémie, syndrome des anticorps antiphospholipides (APL), syndrome thrombotique associé à des anticorps anticardiolipine (AAC) et syndrome anticoagulant lupique (AL),
et/ou où la cystéamine, la cystamine ou le sel pharmaceutiquement acceptable de l'une quelconque de celles-ci est une cystéamine ou un sel pharmaceutiquement acceptable de celle-ci.

3. Cystéamine, cystamine ou sel pharmaceutiquement acceptable de celles-ci pour une utilisation selon la revendication 1 ou la revendication 2, où la cystéamine, la cystamine ou le sel pharmaceutiquement acceptable de l'une quelconque de celles-ci est à une quantité efficace pour réduire le risque de lésion ischémique résultant d'une thrombose.

4. Cystéamine, cystamine ou sel pharmaceutiquement acceptable de l'une quelconque de celles-ci pour une utilisation selon la revendication 2 ou la revendication 3, où le sujet est dans l'une des situations suivantes : cancer, maladie myéloproliférative, myélome multiple, chirurgie, traumatisme, immobilisation, utilisation de contraceptifs oraux, administration de thalidomide ou de ses congénères, éventuellement en combinaison avec un stéroïde, thrombocytopénie induite par l'héparine, grossesse, maladie intestinale inflammatoire, syndrome néphrotique, hémoglobinurie paroxystique nocturne, syndrome d'hyperviscosité ou macroglobulinémie de Waldenström.

5. Cystéamine, cystamine ou sel pharmaceutiquement acceptable de l'une quelconque de celles-ci pour une utilisation selon l'une quelconque des revendications précédentes, où la lésion ischémique est le résultat d'une lésion tissulaire, d'une maladie ou d'un accident vasculaire cérébral ou où la lésion est une lésion de reperfusion, de préférence où la lésion de reperfusion est un résultat d'une chirurgie ou d'une transplantation d'un organe ou où la maladie est la drépanocytose.

6. Cystéamine, cystamine ou sel pharmaceutiquement acceptable de l'une quelconque de celles-ci pour une utilisation selon l'une quelconque des revendications précédentes, où la cystéamine, la cystamine ou le sel pharmaceutiquement acceptable de l'une quelconque de celles-ci est administré avant, en même temps ou après un événement/une crise ischémique.

7. Cystéamine, cystamine ou sel pharmaceutiquement acceptable de l'une quelconque de celles-ci pour une utilisation selon l'une quelconque des revendications précédentes, où la cystéamine, la cystamine ou le sel pharmaceutiquement acceptable de l'une quelconque de celles-ci est formulé pour être administré par voie orale et éventuellement où la cystéamine, la cystamine ou un sel pharmaceutiquement acceptable de l'une quelconque de celles-ci présente un enrobage gastrorésistant, où l'enrobage gastrorésistant est un enrobage de préférence sélectionné parmi les suivants : gélatine polymérisée, gomme laque, copolymère de l'acide méthacrylique de type C NF, butyrate-phtalate de cellulose, hydrogénophtalate de cellulose, propionate-phtalate de cellulose, acétate-phtalate de polyvinyle (PVAP), acétate-phtalate de cellulose (CAP), acétate-trimellitate de cellulose (CAT), phtalate d'hydroxypropylméthylcellulose, acétate d'hydroxypropylméthylcellulose, succinate de dioxypropylméthylcellulose, carboxyméthyléthylcellulose (CMEC), acétate-succinate d'hydroxypropylméthylcellulose (HPMCAS) et polymères et copolymères de l'acide acrylique typiquement formés à partir d'acrylate de méthyle, d'acrylate d'éthyle, de méthacrylate de méthyle et/ou de méthacrylate d'éthyle et de copolymères d'esters de l'acide acrylique et méthacrylique.

8. Cystéamine, cystamine ou sel pharmaceutiquement acceptable de l'une quelconque de celles-ci pour une utilisation selon l'une quelconque des revendications 1 à 6, où la cystéamine, la cystamine ou un sel pharmaceutiquement acceptable de l'une quelconque de celles-ci est formulé pour être administré par voie intrapéritonéale ou est administré par voie intravasculaire.

9. Cystéamine, cystamine ou sel pharmaceutiquement acceptable de l'une quelconque de celles-ci pour une utilisation selon l'une quelconque des revendications précédentes, où l'utilisation comprend une augmentation de la production d'un multimère de bas poids moléculaire (BPM) de l'adiponectine.

10. Cystéamine, cystamine ou sel pharmaceutiquement acceptable de l'une quelconque de celles-ci pour une utilisation selon l'une quelconque des revendications précédentes, où l'utilisation comprend une augmentation du rapport adiponectine de plus bas poids moléculaire (BPM)/adiponectine de poids moléculaire intermédiaire et/ou élevé chez le sujet, éventuellement où le sujet est obèse et présente des taux d'adiponectine de BPM faibles par comparaison à des sujets sains.

11. Cystéamine, cystamine ou sel pharmaceutiquement acceptable de celles-ci pour une utilisation selon l'une quelconque des revendications précédentes, où le sujet a un facteur de risque pour l'ischémie sélectionné parmi les suivants : hypertension, tabagisme, carotidopathie ou autre artériopathie, artériopathie périphérique, fibrillation auriculaire, autre cardiopathie, accidents ischémiques transitoires cérébraux (AIT), une hémopathie, inactivité physique, obésité, consommation excessive d'alcool, utilisation de drogues illégales, antécédents d'accident vasculaire cérébral, anémie drépanocytaire et antécédents de crise cardiaque,
et/ou où le sujet a un événement ischémique dans ses antécédents.

12. Cystéamine, cystamine ou sel pharmaceutiquement acceptable de l'une quelconque de celles-ci pour une utilisation selon l'une quelconque des revendications précédentes, où la cystéamine, la cystamine ou le sel pharmaceutiquement acceptable de l'une quelconque de celles-ci est administré au dit sujet à une concentration dans la plage de 0,5-20 mg/kg de poids corporel,
et/ou où la cystéamine, la cystamine ou le sel pharmaceutiquement acceptable de l'une quelconque de celles-ci est administré dans une dose quotidienne totale de 0,5-4,0 g/m².

13. Cystéamine, cystamine ou sel pharmaceutiquement acceptable de l'une quelconque de celles-ci pour une utilisation selon l'une quelconque des revendications précédentes, où la cystéamine, la cystamine ou le sel pharmaceutiquement acceptable de l'une quelconque de celles-ci est formulé pour être administré avec un ou plusieurs agents additionnels utiles pour le traitement d'une ischémie, de préférence où les un ou plusieurs agents additionnels sont sélectionné parmi un agent de reperfusion, un agent piégeur de radicaux libres et un agent piégeur de spin, un agent neuroprotecteur, un anticoagulant, un antiagrégant plaquettaire, la nimodipine et la naloxone,
et/ou où la cystéamine, la cystamine ou le sel pharmaceutiquement acceptable de l'une quelconque de celles-ci est formulé pour être administré moins de quatre fois par jour, de préférence pour être administré deux fois par jour,
et/ou où la cystéamine, la cystamine ou le sel pharmaceutiquement acceptable de l'une quelconque de celles-ci est formulé pour être administré en continu sur une période comptée en jours, semaines ou mois.

14. Cystéamine, cystamine ou sel pharmaceutiquement acceptable de l'une quelconque de celles-ci pour une utilisation selon l'une quelconque des revendications précédentes formulé pour être administré avec un ou plusieurs agents additionnels utiles pour le traitement d'une maladie thrombotique, éventuellement un anticoagulant, une héparine, un antagoniste de la vitamine K, des dérivés de la 4-hydroxycoumarine, la warfarine, l'acénocoumarol, le dicoumarol, le biscoumacétate d'éthyle, le phenprocoumone, la streptokinase, l'urokinase, un activateur tissulaire du plasminogène (tPA), l'altéplase (tPA recombinant), le rétéplase, le ténectéplase et l'argatroban.

15. Cystéamine, cystamine ou sel pharmaceutiquement acceptable de l'une quelconque de celles-ci pour une utilisation selon l'une quelconque des revendications précédentes, où la cystéamine, la cystamine ou le sel pharmaceutiquement acceptable de l'une quelconque de celles-ci est administré par voie orale avec une formulation de fer biodisponible et/ou où l'utilisation comprend une réduction d'une inflammation secondaire à l'événement ischémique.
